# EUROPEAN PATENT APPLICATION

(11) **EP 3 318 210 A1**
(43) Date of publication of application: **09.05.2018**
(21) Application number: 16306464.5
(22) Date of filing: 08.11.2016
(51) Int. Cl.: A61B 18/14, A61B 18/00, A61B 5/0472

(54) **A MONITORING SYSTEM FOR ABLATION OF CARDIAC TISSUE IN CLOSE PROXIMITY TO THE AV NODE OF THE HEART AND FOR GENERATING A SENSORIAL ALERT**

(71) Applicant: Cardiomed Consulting Ltd, 0630 Auckland (NZ); Centre Hospitalier Universitaire de Bordeaux, 33404 Talence (FR); UNIVERSITE DE BORDEAUX, 33000 Bordeaux (FR); Institut National de la Santé et de la Recherche Médicale, 75013 Paris (FR)
(72) Inventor: HOOKS, Darren, 6011 Wellington (NZ); MEILLET, Valentin, 33320 Eysines (FR); DUBOIS, Rémi, 33700 Merignac (FR)
(74) Representative: Cabinet Camus Lebkiri

(57) **Abstract**

A monitoring system for ablation of cardiac tissue in close proximity to the AV node of the heart, characterized in that it comprises :
▪ At least one ablation catheter (12) powered by a power source (16);
▪ At least one electrode (13) acquiring an intracardiac electrogram (IEGM), called IEGM electrode ;
▪ At least one set of electrodes (14) acquiring a surface electrocardiogram (ECG), called ECG electrodes ;
▪ At least one calculator (15, 16, 17, 18, 19, 25, 27) and a memory for measuring in real time at least one of (i) signals acquired from the IEGM electrode and the ECG electrodes, and (ii) catheter tip location, in order to automatically generate an estimate of the level of risk of heart block, called Risk index (Ri), said risk index (Ri) activating ta sensorial alert.

## Description

### Technical field

The present invention relates to the field of catheter ablation of cardiac arrhythmia, and specifically enables safer ablation close to the atrioventricular node by alerting the operator in impending risk of heart block.

### Background technology

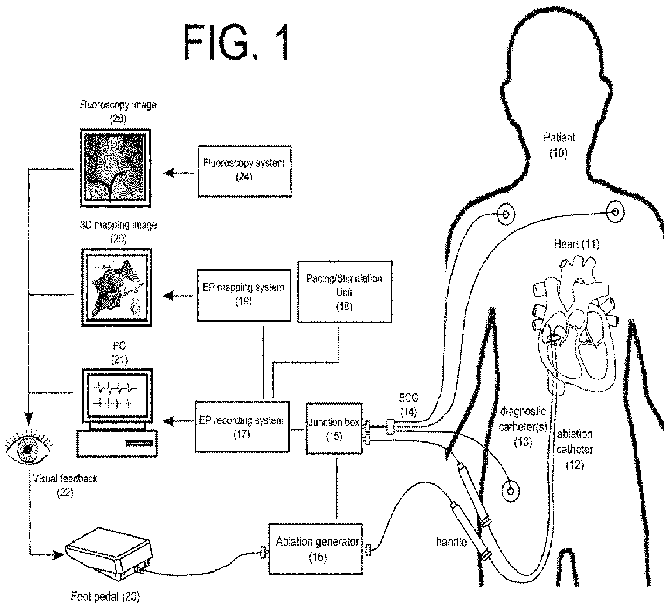

Cardiac arrhythmia occurs when the normal co-ordinated electrical rhythm of the heart is lost. A patient with cardiac arrhythmia may experience palpitations, fatigue, light-headedness, or, in extreme cases, loss of consciousness and death. Many cardiac arrhythmia are amenable to treatment with catheter based ablation. These procedures are typically performed with catheters inserted inside the heart via the femoral veins as represented in figure 1. Ablation energy, typically in the form of radiofrequency RF alternating current, or freezing (cryoablation), is delivered through an 'ablation catheter' 12 via a power source 16, and is used to permanently disrupt/extinguish (ablate) areas of cardiac tissue that are critical to the maintenance of the specific arrhythmia. Ablation catheters are designed to direct ablation energy to the tissue in a focal manner, via conducting tips usually between 4 and 8mm in length and ~2.5mm diameter. The ablation catheter is able to be flexed and rotated to reach all regions of the heart. The power source or generator unit 16 is capable of generating the ablation energy. In the case of RF ablation, current flows from the catheter tip to a return patch electrode on the body surface, causing localised tissue heating and cellular necrosis (death) around the catheter tip. In the case of cryoablation, the catheter tip is cooled initially to ~30°C to assess effect, and then cooled further to ~50°C to -70°C to cause permanent cellular necrosis and tissue disruption. Catheter ablation is an increasingly common procedure, with an estimated 472 procedures performed per million population in France in 2012, and frequently provides a cure to patients with arrhythmia.

In the normal heart, cardiac cells (myocytes) hold negative charge when at rest. During normal function, each heart beat is preceded by a change in cellular charge, so that cells become positively charged ('depolarised'). This process of depolarisation (or 'activation') travels through cardiac tissue as a self-sustaining electrical impulse, travelling as a wavefront, at speeds of 30-100 cm/s, and triggers the heart muscle to contract.

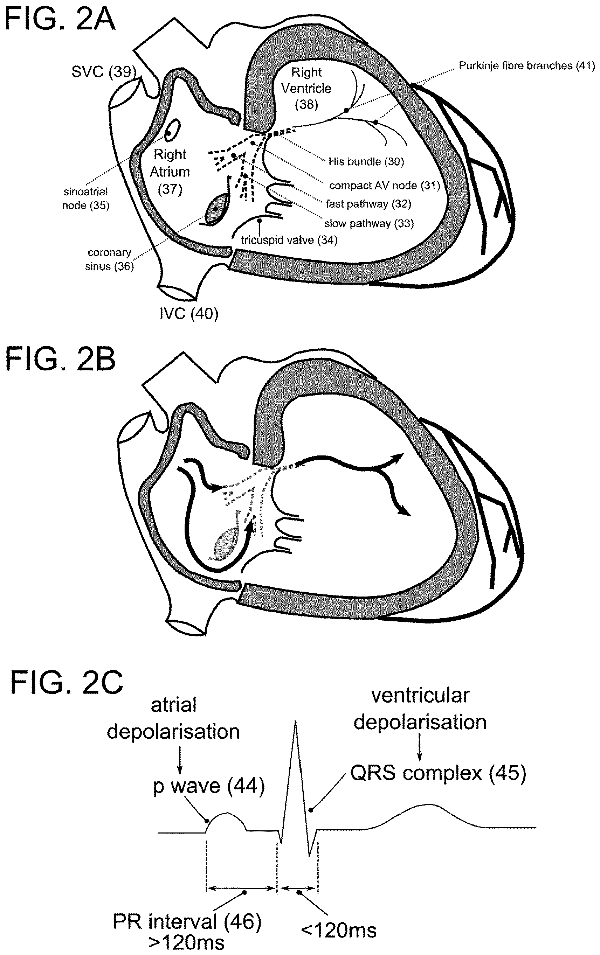

Figure 2A shows a schematic of the heart cut-open to reveal the inside of the right atrium 37 and right ventricle 38 (cut surfaces shown in grey). The right atrium and right ventricle are separated by the tricuspid valve 34.

### AV node

Figure 2A demonstrates the essential components of the electrical function of the heart. The electrical impulse originates in the sinoatrial node 35 (the pacemaker) located in the right atrium and propagates, as shown in figure 2B, through the atria to reach the atrioventricular node. The atrioventricular node is called the "AV node". In the normal case, the AV node is the sole electrical connection between the atria (upper chambers of the heart) and ventricles (lower chambers of the heart), and conducts the impulse to the ventricles. The AV node acts as a temporal filter, limiting the rate at which the ventricles can be electrically stimulated, which protects the ventricles against excessively fast atrial rhythms. As the sole connection between atria and ventricles in the normal heart, the AV node allows co-ordination of atrial and ventricular contraction.

### Atrial side : Slow pathway, fast pathway

On the atrial side, the AV node has a diffuse structure into which the electrical impulse enters. This structure consists of limbs that display varying conduction speeds. These limbs are commonly known as the fast pathway 32 and slow pathway (33), as shown in figure 2A. However there may be more or less than two physiologically distinct limbs, and the limbs represent functional rather than anatomically defined inputs.

### Ventricular side : His bundle, Purkinje fibre branches

On the ventricular side, the conducting limbs of the AV node coalesce to form a more discrete structure, known as the compact AV node 31, which funnels the propagating impulse to a cable-like structure known as the "His bundle" 30 which directs depolarisation to the ventricles, via the Purkinje fibre branches 41. Both the His bundle 30 and Purkinje fibres 41 support a high conduction velocity, enabling rapid propagation of the electrical impulse towards and throughout the ventricular tissue.

### IEGM electrodes

The process of cardiac tissue depolarisation gives rise to electrical potential that can be recorded on electrodes placed inside the heart as so-called 'intracardiac electrograms (IEGMs)'. Such electrodes may be embedded into catheters placed into either the atria, coronary sinus, or ventricles. Electrograms recorded inside the heart are typically of 1-10 milliVolts (mV) in amplitude.

### ECG electrodes

Cardiac tissue depolarisation also gives rise to electrical potential on the body surface, which is recorded on the surface electrocardiogram called ECG and shown in figure 2C. Atrial tissue depolarisation causes a small voltage signal known as the p wave 44, whereas ventricular tissue depolarisation leads to generation of a larger electrical potential on the body surface, known as the QRS complex 45, which is usually 1-5 mV in amplitude.

### Measurement of the A-V interval

When ventricular depolarisation occurs via impulse propagation through the rapidly conducting fibres of the His bundle 30 and Purkinje branches 41, the QRS complex is of narrow morphology, meaning typically < 120ms in width.

Conversely, when ventricular depolarisation arises from an abnormal focus within the ventricular tissue itself, the QRS complex becomes widened, specifically > 120ms. The interval between onset of the p wave and onset of the QRS complex is known as the PR interval 46, and indicates the time taken for the electrical impulse to traverse between atria and ventricle.

Alternatively, the PR interval can be assessed in surrogate fashion by the interval between atrial and ventricular intracardiac electrogram recordings (described as the A-V interval), or by using the atrial intracardiac electrogram and the surface ECG QRS complex.

### Accessory pathway

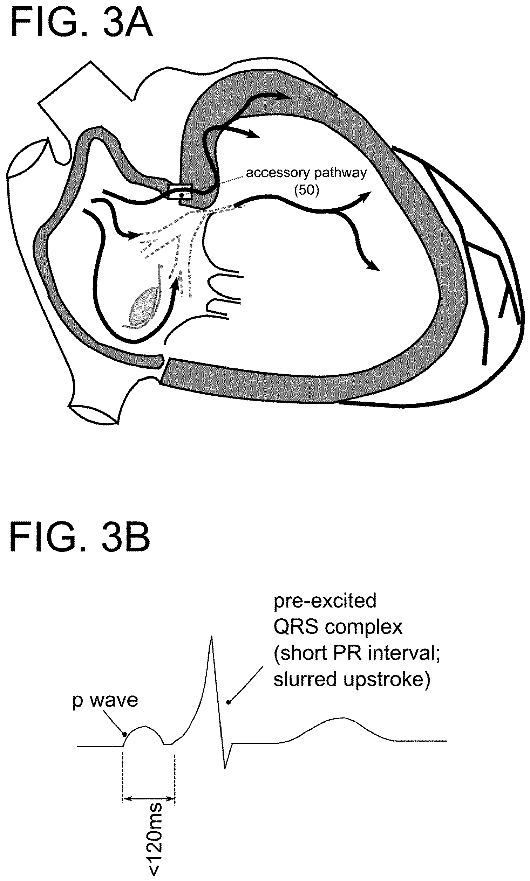

In < 1% of humans, an abnormal connection termed 'accessory pathway' 50, shown in figure 3A, is present between the atria and ventricles. In that case, the electrical impulse is able to travel from atria to ventricles not only via the AV node, but also via the accessory pathway. The presence of an accessory pathway may alter the morphology of the normal QRS complex 45, making it wider and with a slurred upstroke. Figure 3B depicts such a QRS complex, referred to as a 'pre-excited' QRS. In addition, the normal PR interval 46 may be shorted to become < 120ms as represented in figure 3B. Individuals with a pre-excited QRS are said to have the Wolff-Parkinson-White syndrome, and in rare cases are prone to sudden cardiac death, due to loss of the temporal filtering properties of AV node conduction.

### Arrhythmia / AVNRT & AVRT

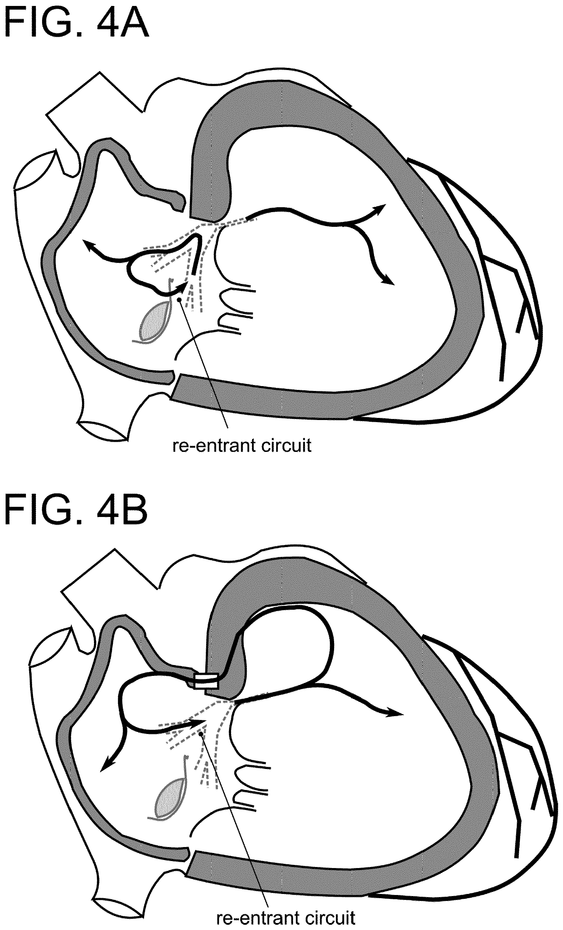

In normal ('sinus') heart rhythm, activation of the heart proceeds in ordered fashion from the atria to the ventricles as shown in figure 2B and 3A. Arrhythmia occurs when there is disturbance of the normal sequence of spread of electrical impulse. Often, arrhythmia is caused by 're-entry', where impulse propagation re-enters previously depolarised (activated) tissue in a circular fashion, causing repeditive and rapid activation of tissue, which leads to sudden elevation of heart rate. For re-entry to occur, a circuit of cardiac tissue is required, typically around an anatomical or functional obstacle, through which the impulse wave is unable to propagate. This circuit can be formed by the two limbs of the AV node, in which case the arrhythmia is termed atrioventricular nodal re-entrant tachycardia called AVNRT and shown in figure 4A. Because these limbs have different functional (electrical) properties, they are apt to sustain re-entry in susceptible persons. Alternatively, in persons with the Wolff-Parkinson-White syndrome, the re-entrant circuit may be formed by the AV node and an accessory pathway, in which case the arrhythmia is termed atrioventricular reciprocating tachycardia called AVRT and shown in figure 4B.

### Ablation for AVNRT / Slow pathway

Ablation for AVNRT is typically performed over the slow pathway of the AV node 33 to disrupt or modify the slow pathway to an extent that re-entrant impulse propagation is no longer possible.

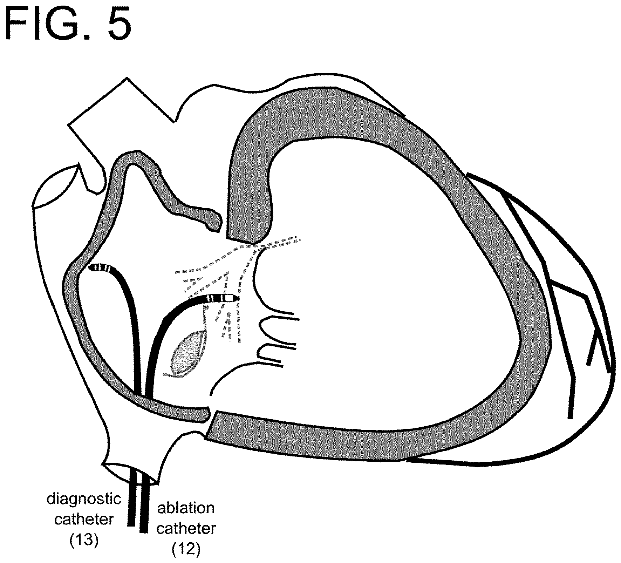

Figure 5 depicts ablation over the slow pathway 33 using an ablation catheter 12, with an additional diagnostic catheter 13 placed in the position of the high right atrium.

### Ablation for AVRT / Accessory pathway

Ablation for AVRT is typically performed over the accessory pathway 50, which may lie anywhere along the valve planes that separate the atria and ventricles. In some cases, the accessory pathway 50 may be close, or adjacent, to the AV node, lying on the inter-atrial septum, as demonstrated in FIG. 3A. In these cases it is described as being located in mid- or antero- septal location. Ablation of a mid- or antero- septal accessory pathway may be undertaken for the purposes of cure of AVRT, or for the prevention of sudden cardiac death in individuals with the Wolff-Parkinson-White (WPW) syndrome.

Long-term cure rates of AVNRT with a single ablation procedure are ∼95%. Ablation of mid- or antero- septal APs is successful in approximately 80% of cases with a single procedure.

### Ablation procedure : Two catheters needed

Catheter ablation is generally performed following a diagnostic electrophysiology study, in which the mechanism of arrhythmia is determined. Such diagnostic study generally requires a minimum of two catheters, one of which may be the ablation catheter 12.

One catheter is placed in the right atrium or coronary sinus to record an atrial intra-cardiac electrogram A IEGM. Another catheter is placed alternately between the region of the His bundle to record a His electrogram, and the right ventricular apex to record a ventricular intra-cardiac electrogram V IEGM. Additional catheters may also be placed according to operator preference to allow the simultaneous recording of IEGMs in more locations.

During the diagnostic study, the heart rhythm is manipulated (stimulated) by pacing protocols in ways that define the arrhythmia mechanism. In general, programmed stimulation is required from both the atria and the ventricles in order to define the mechanism of arrhythmia. During ablation of AVNRT, AVRT, or a septal accessory pathway, a minimum of two catheters is generally required, one being the ablation catheter, and the other being the catheter recording an atrial intra-cardiac electrogram. The atrial intra-cardiac electrogram is required primarily because atrial electrical activity is not always well seen on the body surface ECG, due to atrial activity being of smaller amplitude compared to ventricular activity. At times, it is also necessary to perform atrial pacing during ablation.

### Risks of the ablation procedure

Catheter ablation of arrhythmia is generally a well-tolerated procedure., however, carries a degree of risk. Specifically, ablation of either AVNRT, or a mid- or antero- septal accessory pathway, risks damage to the compact AV node or His bundle due to the proximity of the ablation catheter to these structures as shown in figure 5.

### AV block

Damage to the compact AV node or His bundle can cause heart block to occur, hereafter referred to as `AV block'. AV block describes a condition where conduction of electrical impulses between the atria and ventricles is limited or completely extinguished. Heart block leads to a loss of co-ordination between the atria and the ventricles, and can lead to a lowering of ventricular heart rate, loss of blood pressure, loss of consciousness, or even death.

### Pacemaker

Where damage to the compact AV node or His bundle causes permanent AV block, implantation of a permanent pacemaker is usually required to restore atrial and ventricular synchrony. Permanent pacemakers carry significant morbidity due to the necessity of further surgery for generator changes every 5 to 12 years, and the possibilities of generator or lead failure, infection requiring removal, and detrimental long-term effects of pacing on ventricular function. It is therefore highly desirable to minimise the risk of AV block when performing ablation close to the AV node. Currently, as many as 26% of cardiac ablation procedures are for AVNRT. With current ablation equipment and techniques, the complication of AV block and pacemaker insertion occurs in between 0.5-1% of cases.

Ablation for mid- or antero- septal AP comprises < 5% of all ablation procedures, however, the risk of AV block is higher than for AVNRT, and is estimated at about 2-5%.

Ablation of AVNRT or AVRT is generally performed during normal (sinus) heart rhythm, however may be performed during tachy-arrhythmia.

### Cardiac rhythm

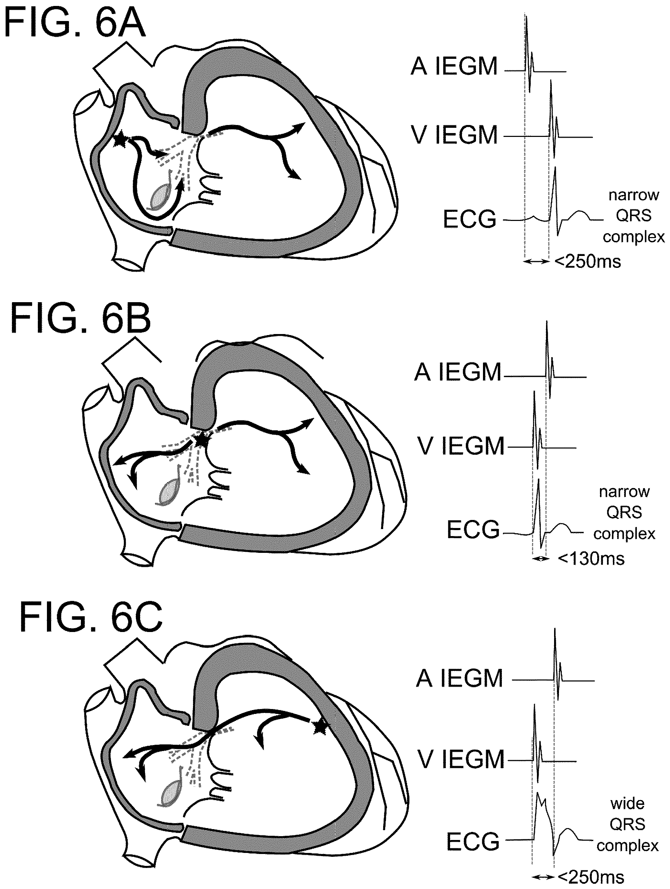

During ablation, heart rhythm may be expected to fluctuate between sinus rhythm as shown in figure 6A, junctional rhythm as shown in figure 6B, or ventricular ectopic rhythm as shown in figure 6C.

In sinus rhythm, if AV node function is normal, each atrial depolarisation, as seen on the atrial intracardiac electrogram A IEGM, should be followed within ∼250ms by a ventricular depolarisation, as seen either on a ventricular intracardiac electrogram V IEGM, or, on the surface ECG as a QRS complex, as shown in figure 6A.

Junctional rhythm is caused by heating to the slow pathway 33 and compact AV node 31 and/or His bundle 30, and describes a concentrically propagating wavefront emanating from the AV node or His bundle and causing near simultaneous atrial and ventricular activation, such that atrial depolarisation and ventricular depolarisation occur within ∼130ms of each other.

In both sinus and junctional rhythms, activation of the ventricles is via the His bundle 30 and Purkinje fibres 41, and accordingly the QRS complex on the ECG is of narrow type as shown in figures 6A and 6B, excluding conditions of disease to the Purkinje fibres.

Ventricular ectopic rhythm describes activation commencing in the ventricular tissue, with retrograde AV nodal conduction to the atria, as illustrated in figure 6C, such that each atrial depolarisation should lag ventricular depolarisation by no more than ∼ 250ms. Ventricular ectopic rhythm can occur spontaneously, or be induced by the presence of a catheter in the ventricle, in which case it is caused by the mechanical action of the catheter against the ventricular wall. In the case of ventricular ectopic rhythm, activation of the ventricles is not via the His and Purkinje fibres, and accordingly the QRS complex of the ECG is widened and of different morphology to normal as shown in figure 6C. The timing limits of 130ms and 250ms described here are a guide, and may differ based on exact location of intracardiac catheters or placement of ECG leads, and the heart rate and physiology of the individual patient. Nonetheless, absolute timings prove a reliable method of determining abnormal electrical impulse conduction in the AV node.

### Indicators of AV block

AV block caused by ablation is often heralded by changes in the expected cardiac rhythms described above. There may be transient loss of atrio-ventricular association, such that each atrial depolarisation is not followed by a ventricular depolarisation, or vice versa, or the time limits for atrial or ventricular depolarisation outlined above are exceeded. In other cases, excessively rapid junctional rhythm (>130 beats per minute) may indicate impending AV block. In yet other cases, a prolongation of the normal PR interval 46 may indicate imminent AV block.

AV block may also be heralded by sudden displacement of the ablation catheter tip away from the desired location of ablation, towards the compact AV node 31 and/or His bundle 30. Such displacement can be detected radiographically by continuous low-intensity x-ray (fluoroscopy) of the catheter, or, via the monitoring of catheter tip location with 3-dimensional mapping systems 19 which typically use impedence and/or magnetic tracking methods to localise the catheter tip.

The risk of causing heart block varies according to the which of the above conditions is identified. For example, rapid junctional rhythm in isolation may portend less risk than PR interval lengthening. In another example, rapid junctional rhythm in isolation may portray less risk compared to the same rhythm combined with loss of atrio-ventricular association. Moreover, a sudden unexpected shift of catheter tip location in itself may herald less risk than a shift combined with change in cardiac rhythm. There is, therefore, a gradation of risk, and the level of risk accepted during ablation will depend on the operator and the patient-specific circumstances of ablation.

### Manual termination of ablation

Currently, the risk of AV block is minimised by close attention of the operator (and ancillary staff) to cardiac rhythm and ablation catheter location during ablation, with manual termination of ablation energy, commonly by use of a foot pedal 20, where risk markers of heart block are identified. There is, therefore, a human visual feedback loop 22, as illustrated in figure 1, where the operator and ancillary staff monitor rhythm and catheter movement and stop ablation if required due to excessive risk. Monitoring of cardiac rhythm is facilitated by the display of intra-cardiac electrograms and the surface electrocardiogram on the EP recording system monitor 21. Monitoring of ablation catheter location is by visual assessment of location based on displays provided by at least one of the fluoroscopy system 24 and the EP mapping system 19.

However, visual monitoring of both cardiac rhythm and catheter location during ablation are complex tasks, each task competing with the needs of the other. In addition, human reaction times to changes of cardiac rhythm and catheter position are variable, often exceed one second, and are frequently longer in trainees learning the art of cardiac ablation. A system to facilitate more rapid and consistent termination of ablation energy in response to indicators of AV block is therefore desirable.

### Prior Art

The US patent No. 7,578,816 B2 (Boveja et al.) is directed toward control of ablation in similar cardiac rhythms. The Boveja application mainly discloses using automated monitoring of cardiac rhythm based exclusively on intracardiac signals, acquired from within both an atrial chamber and a ventricular chamber. This approach suffers several important limitations, which the present invention seeks to address.

Firstly, this solution (Boveja application) requires placement of a third catheter during ablation, namely a ventricular catheter. The use of an additional catheter adds considerable cost to the procedure.

Secondly, presence of a ventricular catheter during ablation significantly increases the burden of ventricular ectopic beats, termed here ventricular ectopic rhythm, as shown in figure 6C. This is related to the mechanical action of the catheter on the ventricular tissue. Although ventricular ectopic rhythm can be reduced by careful catheter placement, it cannot usually be completely eliminated.

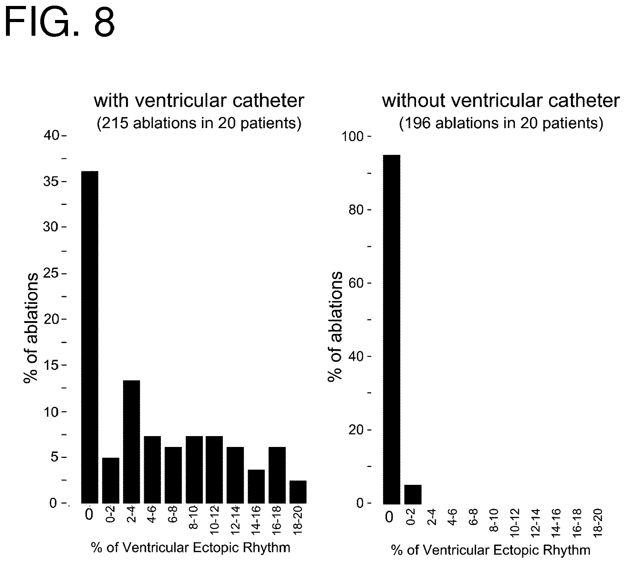

Evidence of such increase in ventricular ectopic rhythm is shown in FIG. 8, which documents the percentage of ventricular ectopic rhythm observed during 215 routine ablations performed in 20 patients, with a ventricular catheter placed by a clinician expert in the field of cardiac ablation, and during 196 ablations performed in 20 patients without a ventricular catheter placed. In the former group, only 36% of ablations were not complicated by ventricular ectopic rhythm, and the remaining 64% of ablations suffered from ventricular ectopic rhythm ranging from 0.1% to 20% of all heart beats. In the latter group, 95% of ablations were not complicated by ventricular ectopic rhythm, and only 5% of ablations suffered from ventricular ectopic rhythm, of <2% of all heart beats.

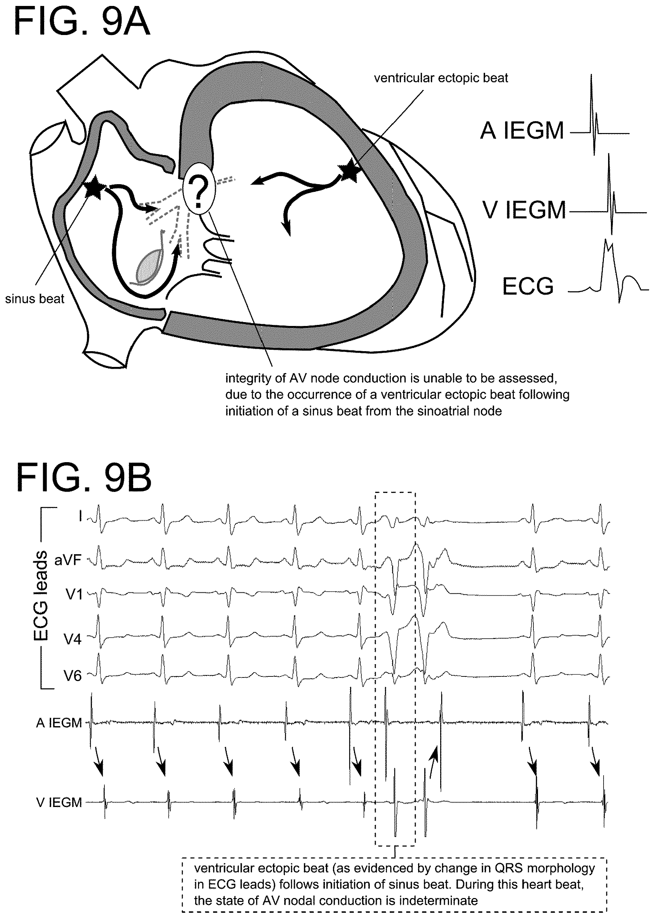

Ventricular ectopic rhythm during ablation is undesirable because it may limit the ability to discern the integrity of AV nodal conduction, and therefore continue ablation in a safe manner. This is because if a ventricular ectopic beat occurs in the period immediately following initiation of a sinus beat in the sinoatrial node 35, the state of AV nodal conduction is indeterminate, meaning that it cannot be determined if the electrical impulse was safely conducted by the AV node, or indeed, if AV block had occurred. The phenomenon of ventricular ectopic rhythm masking the integrity of AV nodal conduction is illustrated in FIG. 9A, and an example of signals acquired from a patient during ablation demonstrating this phenomenon is given in FIG. 9B.

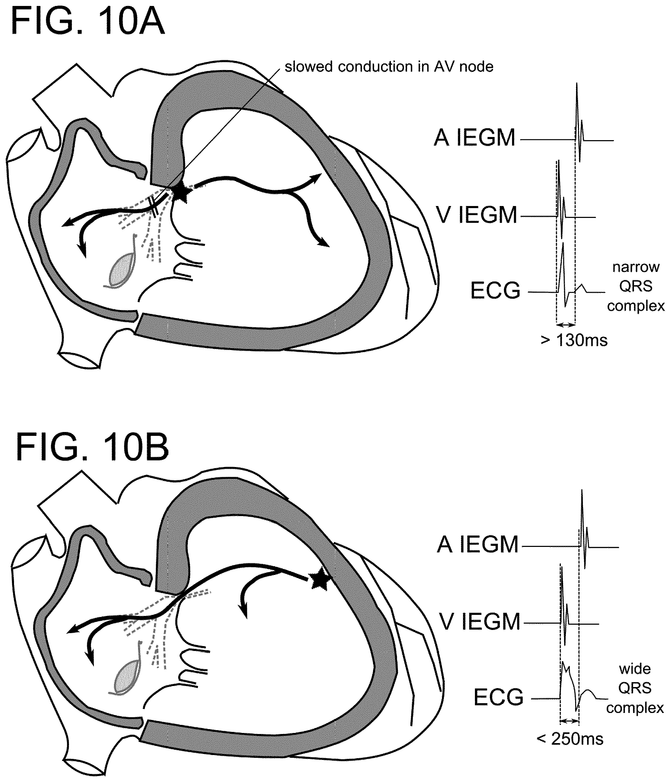

Thirdly, reliance on ventricular intracardiac recordings for discerning cardiac rhythm limits a device's ability to distinguish various cardiac rhythms. For example, such a device is unable to:
(i) differentiate :
   ▪ junctional rhythm with impaired (slowed) AV nodal conduction, ie. atrial depolarisation lagging ventricular depolarisation by > 130ms, indicating impending heart block; as shown in figure 10A, from :
   ▪ ventricular ectopic rhythm with normal AV nodal conduction, ie. atrial depolarisation lagging ectopic ventricular depolarisation by < 250ms, as shown in figure 10B.

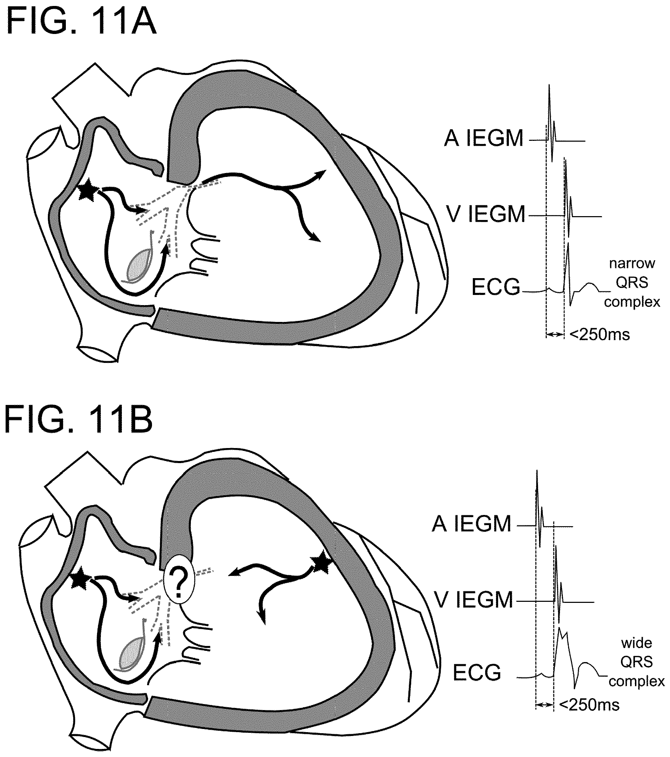

It can be seen from figures 10A and 10B that the intracardiac signals do not substantially differ in timing with each of these two rhythms.
(ii) differentiate :
   ▪ sinus rhythm with normal AV nodal conduction, ie. atrial depolarisation followed by a normal ventricular depolarisation within 250ms, as illustrated in figure 11A, from :
      ▪ sinus rhythm with indeterminate AV nodal conduction due to occurrence of a ventricular ectopic, ie. atrial depolarisation followed by a ventricular ectopic depolarisation within 250ms, as illustrated in figure 11B.

It can be seen from figures 11A and 11B that the intracardiac signals do not substantially differ in timing with each of these two rhythms.
(iii) distinguish pre-excited ventricular activation, as shown in figures 3A and 3B, from normal ventricular activation, as shown in figures 2B and 2C, independent of the speed/integrity of AV nodal conduction. Such distinction is important during the ablation of an accessory pathway.

Another important distinction is that the Boveja application is limited to automated cut-out of ablation via placement of a control switch within the power supply line of the ablation generator, or between the ground patch and the ablation generator unit, or between the generator unit and the ablation catheter. These arrangements all suffer from the limitations inherent in releasing direct control of human therapy to a machine. Without a human decision maker having ultimate responsibility for the application and termination of ablation energy, there is possibility of machine-related harm.

### SUMMARY OF THE INVENTION

One aspect of the invention concerns, a monitoring system for ablation of cardiac tissue in close proximity to the AV node of the heart, in order to improve the safety of ablation.

The monitoring system of the invention comprises :
▪ At least one ablation catheter powered by a power source ;
▪ At least one electrode acquiring an intracardiac electrogram, called IEGM electrode ;
▪ At least one set of electrodes acquiring a surface electrocardiogram, called ECG electrodes ;
▪ At least one calculator and a memory for measuring in real time signals acquired from the IEGM electrode, the ECG electrodes, and the catheter tip imaging or positioning system, in order to automatically generate a level of risk indicator (Risk Index (Ri)), conveyed to the operator via at least one sensorial signal.

One advantage is that this solution informs an operator of the degree of risk and allows rapid discontinuation of ablation.

In one embodiment of the invention, the monitoring system comprises at least one calculator and a memory for measuring in real time a displacement of the ablation catheter tip in order to refine the Risk Index (Ri) when detecting a displacement of the ablation catheter exceeding a predefined limit.

In one embodiment, real time displacement of the ablation catheter away from the desired ablation location is determined from at least one of (i) catheter localisation based on object tracking in fluoroscopic low-dose x-ray images, or (ii) three-dimensional catheter tip position obtained from measurement of impedance and/or magnetic field at the catheter such as performed in an EP mapping system.

In this application we describe such a system, which utilises computational real-time analysis of cardiac rhythm and ablation catheter tip location in order to automatically derive an index of risk of continued ablation, in a near-instantaneous fashion, when markers of risk for AV block are detected.

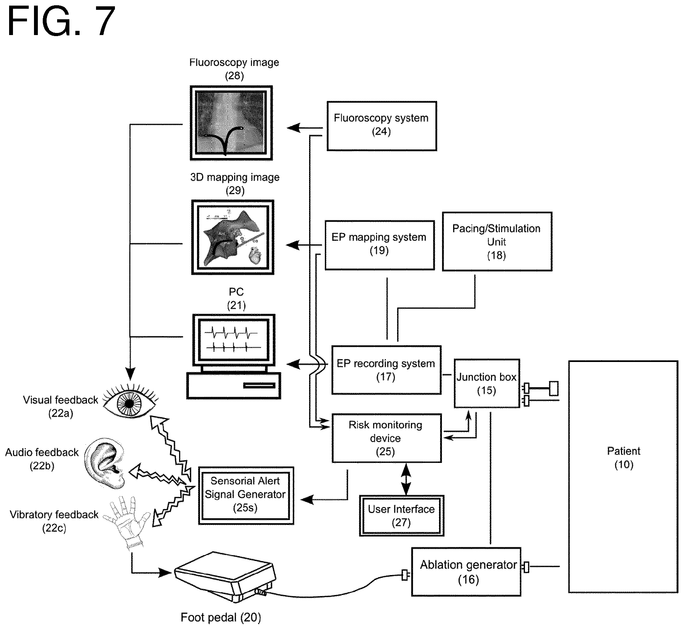

The system of the invention is directed to improvement of the safety of ablation when applied close to the AV node. A system is described in figure 7 that allows real-time or near real-time monitoring of cardiac rhythm and catheter tip location via a Risk Monitoring Device in order to assess rhythm conditions and catheter movements that may herald development of AV block. The system allows notification to the operator via sensorial alerts generated by a Sensorial Alert Signal Generator 25s, when cardiac rhythm or displacement conditions are identified that indicate risk of AV block. Such notification allows faster reaction times and increased consistency compared to human manual assessment of risk.

An advantage of the invention compared to Prior Art is to utilise analysis of the body surface electrocardiogram ECG for determining ventricular depolarisation, in place of a ventricular intra-cardiac electrogram. The system employed incorporates both ECG QRS complex timing and morphology analysis.

In doing so, the system of the invention has the advantages of :
▪ one less catheter required during ablation because the ventricular catheter is not required,
▪ less ventricular ectopic rhythm due to absence of mechanical action of a ventricular catheter on the heart, and ;
▪ enhanced ability to discriminate cardiac rhythm. An outline of the enhanced cardiac rhythm discrimination can be made by reference to figures 10 and 11.

The surface ECG can be used to reliably distinguish the rhythms of figures 10A and 10B based on analysis of QRS complex morphology. In the case of junctional rhythm with impaired (slowed) AV nodal conduction, as shown in figure 10A, the QRS complex morphology is narrow, and ablation should be discontinued. However, in the case of ventricular ectopic rhythm with normal AV nodal conduction as shown in figure 10B, the QRS complex has a widened morphology, and it is safe to continue ablation. Similarly, the surface ECG can be used to reliably distinguish the rhythms of figures 11A and 11B based on analysis of QRS complex morphology. In the case of sinus rhythm with normal AV nodal conduction, as represented in figure 11A, the QRS complex morphology is narrow, and it is safe to continue ablation. However, in the case of sinus rhythm with indeterminate AV nodal conduction due to occurrence of a ventricular ectopic, see figure 11B, the QRS complex morphology is widened, and it may not be safe to continue.

Another advantage of the invention compared to Prior Art is to offer automated assessment of a graduated level of risk of continued ablation. The risk level, called Risk Index Ri, may be calculated as a weighted sum of the number of cardiac rhythm risk conditions together with catheter tip displacement measures. The Risk Index Ri is conveyed to the operator via a combination of sensorial alerts, with at least one alert emitted. These alerts include audio, visual, and vibratory signals which convey the magnitude of risk of continued ablation, one a graded scale from minimal to extreme risk. This allows the operator to make an instant informed decision whether to cease ablation. By keeping the human operator as the end decision-maker with respect to delivery or cut-out of therapy, the risk of machine-related harm is minimised, and ablation is able to be continued in situations of low risk if desired.

In one embodiment, the risk index Ri triggers an audible alert to the operator, projected by way of audio speaker and/or headphones, prompting the operator to discontinue ablation.

In one embodiment, the frequency of the audible alert varies according to the Risk Index Ri.

In one embodiment, the volume of the audible alert varies according to the Risk Index Ri.

In one embodiment, the audible alert is generated within a stand-alone Risk Monitoring Device 25.

In one embodiment, the audible alert is generated within existing equipment such as an ablation generator 16, EP recording system 17, or EP mapping system 19.

In one embodiment, the Risk Index Ri triggers a visual alert to the operator, prompting the operator to discontinue ablation.

In one embodiment, the visual alert is generated on an electronic monitor or screen associated with a stand-alone Risk Monitoring Device 25.

In one embodiment, the visual alert is generated on an electronic monitor or screen associated with existing equipment such as an ablation generator 16, EP recording system 17, or EP mapping system (19).

In one embodiment, at least one of colour, size, or pattern of the visual alert varies according to the Risk Index Ri.

In one embodiment, the Risk Index Ri triggers a vibratory alert to the operator, prompting the operator to discontinue ablation.

In one embodiment, the vibratory alert comprises vibration of the ablation catheter handle.

In one embodiment, the vibratory alert comprises vibration of the ablation foot pedal.

In one embodiment, at least one of the intensity or pattern of vibratory alert varies according to the Risk Index Ri.

In one embodiment, the system is configured to generate a unique combination of sensorial alerts depending on the Risk Index Ri which indicates the level of risk of heart block. The alert combination may comprise any combination of audio, visual, or vibratory alerts, using either synchronised or sequential timing.

In one embodiment, a first Risk Index RI1 may activate one visual alarm with an appropriated display, a second Risk Index RI2 higher than the first Risk Index may activate a specific sound with an appropriated headphone. A third Risk Index RI3 higher than the second one RI2 may activate the visual alarm and the specific sound with a higher volume.

Others combinations of different Risk Index levels may be take into account the strategy of activating different sensorial signals.

Advantageously, the monitoring system may be achieved by the integration of a calculator or a set of calculators into one or more of an electrophysiology EP recording system 17, an electrophysiology EP mapping system 19, a Pacing/Stimulation Unit 18, a junction box 15, an ablation generator 16 or into a stand-alone Risk Monitoring Device 25.

Advantageously, the monitoring system may be achieved by the integration of a memory or a set of memories into one or more of an electrophysiology EP recording system 17, an electrophysiology EP mapping system 19, a Pacing/Stimulation Unit 18, a junction box 15, an ablation generator 16 or into a stand-alone Risk Monitoring Device 25.

In one aspect of the invention, the calculator comprises a signal timing function in order to derive timing of atrial and ventricular depolarisation, the atrial depolarisation being measured by the IEGM electrode, and the ventricular depolarisation being measured by the ECG electrodes.

In one embodiment, the signal timing function incorporates a voting function allowing the determination of signal timing when more than one IEGM or more than one ECG signal is available to characterise depolarisation timing. One advantage is to avoid false detections of signal timing.

In one embodiment, the monitoring system comprises only one catheter which comprises at least one electrode allowing the acquisition of an intracardiac electrogram, as the IEGM electrode.

In one embodiment, the signal timing function compares the delay between atrial and ventricular depolarisations to a minimum predefined threshold of time and/or a maximum predefined threshold of time. The signal timing function is configured for contributing to the Risk Index Ri when said measured delay exceeds at least one of the predefined thresholds.

In one embodiment, the calculator comprises a QRS complex signal morphology function in order to determine the nature of the ventricular depolarisation as being via the His bundle and Purkinje fibre branches , or, via a ventricular ectopic, or, via an accessory pathway.

In one embodiment, the signal morphology function compares an acquired QRS complex signal with one or more template QRS complexes stored in a memory, the comparison being processed using an auto-correlation function or a cross-correlation function.

In one embodiment, the signal morphology function is configured to compute the QRS complex width and/or to determinate deflection sequence of the complex QRS signal.

In one embodiment, the signal morphology function incorporates a voting function allowing the determination of signal morphology when more than one ECG signal is available to characterise depolarisation morphology. One advantage is to avoid false detections of signal morphology.

In one embodiment, the calculator is configured to characterise the real time or near real time cardiac rhythm given return values of the signal timing function and/or a return value of the signal morphology function. One advantage is to accurately define cardiac rhythm and detect rhythms which indicate a risk of AV block, allowing the automatic cut-out of ablation power.

In one embodiment, the cardiac Risk Index Ri is a probability of AV block occurrence, said probability being characterised by at least one occurrence of :
▪ an atrio-ventricular interval prolongation ;
▪ a ventriculo-atrial interval prolongation ;
▪ an atrio-ventricular dissociation ;
▪ a ventriculo-atrial dissociation ;
▪ a fast junctional rhythm ;
▪ a PR interval prolongation ;
▪ a consecutive atrial depolarisations ;
▪ a consecutive ventricular depolarisations, or ;
▪ an indeterminate atrio-ventricular nodal conduction.
▪ displacement of the ablation catheter tip exceeding a pre-defined limit away from the desired ablation location.

In one embodiment, the memory allows storage of data acquired by the electrodes, storage of QRS complex templates for the purpose of morphology analysis, and storage of predefined parameters such as the cardiac rhythms and ablation catheter tip displacements deemed to indicate risk of AV block.

In one embodiment, the calculator comprises a mapping function allowing the real-time or near real-time automatic tracking of the location of the tip of the ablation catheter.

In one embodiment, the location of the catheter tip is determined in three-dimensions by data provided by a method for catheter tracking based on impedance or magnetic measurements such as those used in an EP mapping system 19.

In one embodiment, the location of the catheter tip is determined in two or three dimensions by analysis of catheter tip movement based on object tracking using low dose x-ray (fluoroscopy) images.

In one embodiment, the calculator is configured to account for the type of ablation procedure, being one of AVNRT, AVRT, or accessory pathway (50) ablation. One advantage is to allow tolerance of different cardiac rhythms or the setting of different AV node conduction time limits depending on the type of ablation being performed.

In one embodiment, the calculator comprises a signal amplification function, a filtering function, an analog to digital conversion function, and computational analysis functions of signal timing and signal morphology. One advantage is elimination of noise from the IEGM and ECG signals, and conversion of signals to digital format thereby allowing digital processing by the signal timing and morphology functions.

In one embodiment, the monitoring system comprises a foot pedal 20 for manually switching off the power source 16 of the ablation catheter 12 via foot pedal release

In one embodiment, the monitoring system comprises a user interface allowing the selection of :
▪ a first set of parameters as inputs to the signal timing function, and/or;
▪ a second set of parameters as inputs to the signal morphology function, and/or;
▪ a third set of parameters for processing the location of the ablation catheter tip, and/or;
▪ a fourth set of parameters for defining the cardiac rhythms of risk which will contribute to the Risk Index Ri ;
▪ a fifth set of parameters for defining the ablation catheter displacement limit which when exceeded will contribute to the Risk Index Ri.

One advantage is to account for a specific patient's physiology, such that tolerance to various cardiac rhythms and/or atrio-ventricular conduction delays can be incorporated to avoid false positive cut-outs of ablation energy. Yet a further advantage is to allow for operators of various experience level, ranging from novice operators for whom cut-out parameters will be stringently set, to experienced operators for whom some cut-out parameters may be relaxed.

In one embodiment, the monitoring system comprises a screen displaying pulse markers in real time, the pulse markers corresponding to a onset or a peak of each electrophysiology recording acquired by the IEGM electrode and the ECM electrodes. One advantage is to allow an operator a visual check of monitoring system integrity.

In one embodiment, the monitoring system comprises a pacing unit that provides current pulses at programmed rates and patterns for stimulating the heart.

In one embodiment, the monitoring system comprises a junction box that provides a common interface gathering the connections of ECG electrodes and IEGM electrode and delivers the acquired signals to the calculator.

Another object of the invention concerns a monitoring method achieving automatically the functions supported by the calculator of the system of the invention.

In one embodiment, the monitoring method increases safety of cardiac ablation when applied in close proximity to the AV node, by automated assessment of risk in response to cardiac rhythm related markers of risk for heart block, comprising the steps of:
▪ Monitoring in real time or near real time, one or more atrial intracardiac signals and the body surface electrocardiogram signals of a patient, wherein said monitoring comprises the steps of signal amplification, filtering, analog to digital conversion, and computational analysis of signal timing and morphology.
▪ Detecting in real time or near real time, an event that may herald imminent heart block, wherein said event is a condition tailored to the location of ablation and arrhythmia type being ablated, and said event may be one of atrio-ventricular dissociation, ventriculo-atrial dissociation, fast junctional rhythm, any junctional rhythm, atrio-ventricular interval prolongation, PR interval prolongation, consecutive atrial depolarisations, consecutive ventricular depolarisations, or indeterminate atrio-ventricular nodal conduction;
▪ Notifying the operator of the level of risk via a single or combined sensorial alert, being at least one of an audio, visual, or vibratory signal

In one embodiment, computational analysis of signal timing comprises methods of determining onset or peak of electrogram depolarisation deflection, and electrocardiogram QRS complex.

In one embodiment, computational analysis of signal morphology comprises methods of determining the extent of electrocardiogram QRS complex match to a template QRS complex signal, and methods of determining electrocardiogram QRS complex width and sequence of deflections.

In one embodiment, computational analysis can be written using development tools and graphical programming applications or languages such as LAB WINDOWS®/CVI, LABVIEW®, MICROSOFT VISUAL BASIC®, MICROSOFT VISUAL C++®, or functionally equivalent software

In one embodiment, the results of computational analysis of signal timing are displayed in real-time or near real-time, in the form of pulse markers, on an electrophysiology recording system monitor, to allow a visual check of said computational analysis integrity.

In one embodiment, the method may be incorporated into or combined with or embedded with an ablation generator.

In one embodiment, the method may be incorporated into or combined with or embedded with an electrophysiology recording system.

In one embodiment, the method may be incorporated into or combined with or embedded with an electrophysiology mapping system.

In one embodiment, ablation is performed for atrio-ventricular nodal re-entrant tachycardia (AVNRT), or for mid-septal or antero-septal accessory pathway ablation.

In one embodiment, ablation is one of: radiofrequency RF ablation, cryoablation, high intensity focused ultrasound HIFU ablation, or microwave ablation.

In one embodiment, atrio-ventricular dissociation refers to an atrial depolarisation without a corresponding ventricular depolarisation within a given time limit.

In one embodiment, ventriculo-atrial dissociation refers to a ventricular depolarisation without a corresponding atrial depolarisation within a given time limit.

In one embodiment, a user interface is provided to the operator or ancillary staff in order to alter settings and parameters which trigger said automatic stopping of energy delivery, based on the type and location of ablation being performed, the nature of the patient's physiology, and operator personal preference.

### BRIEF DESCRIPTION OF THE DRAWINGS

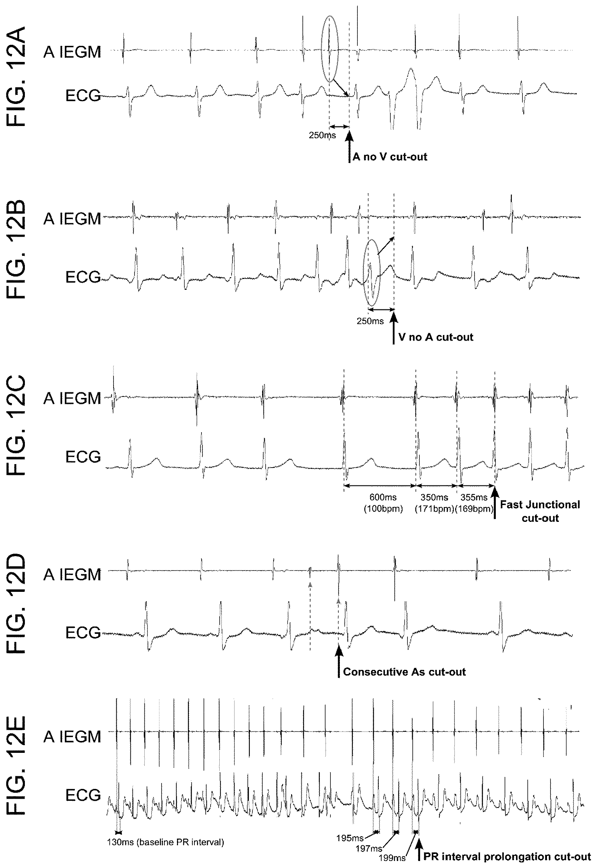

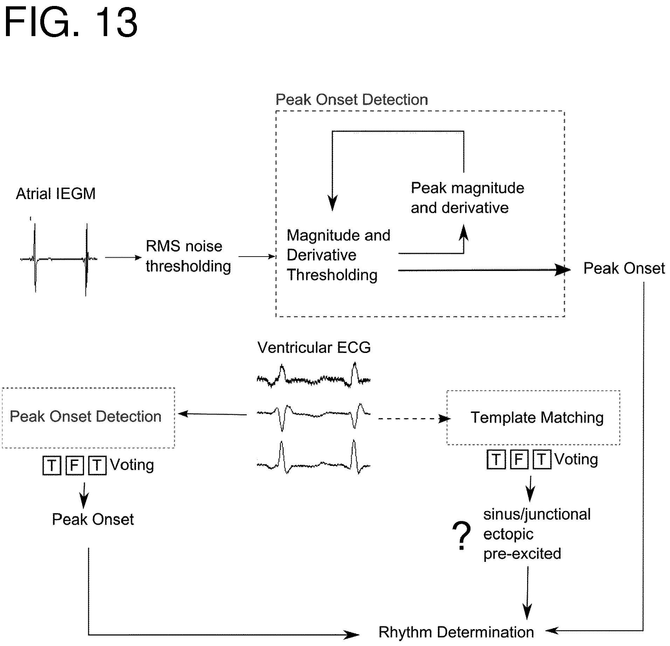

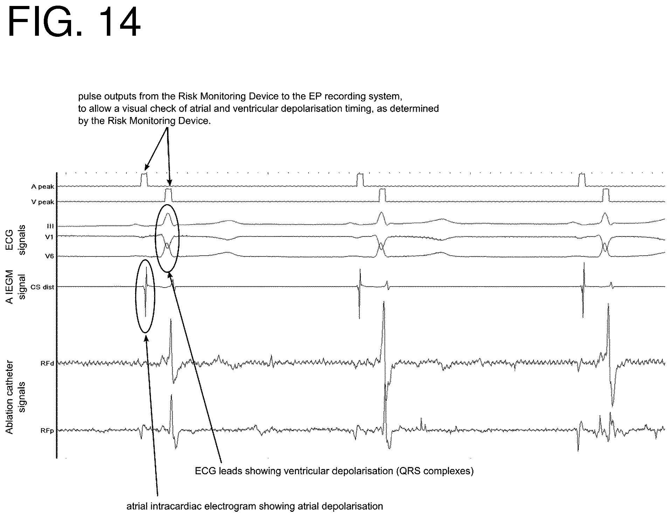

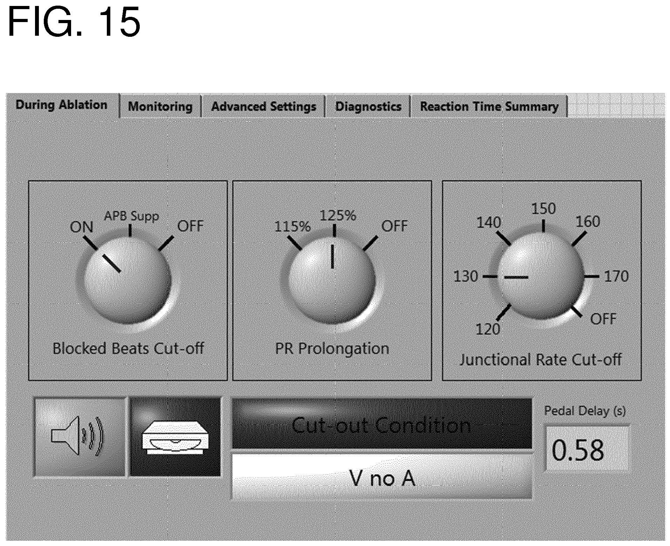

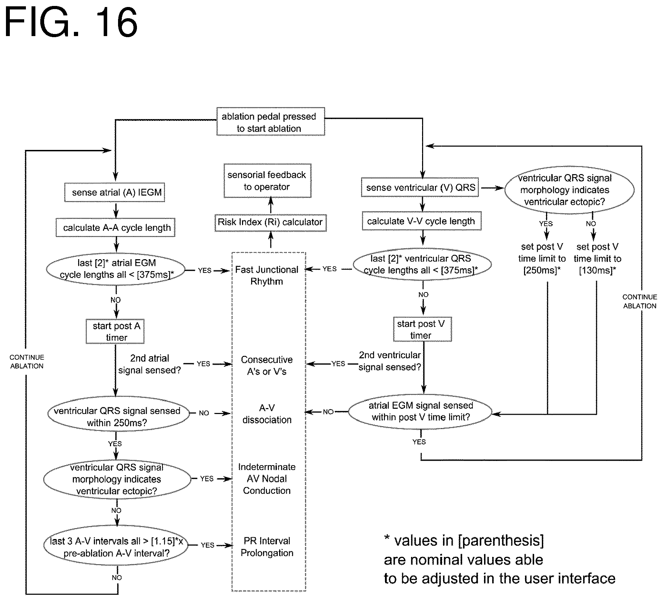

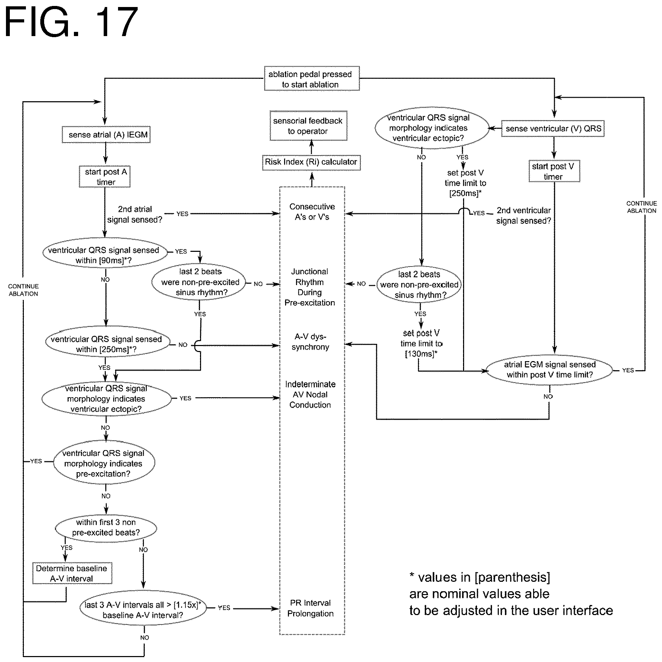

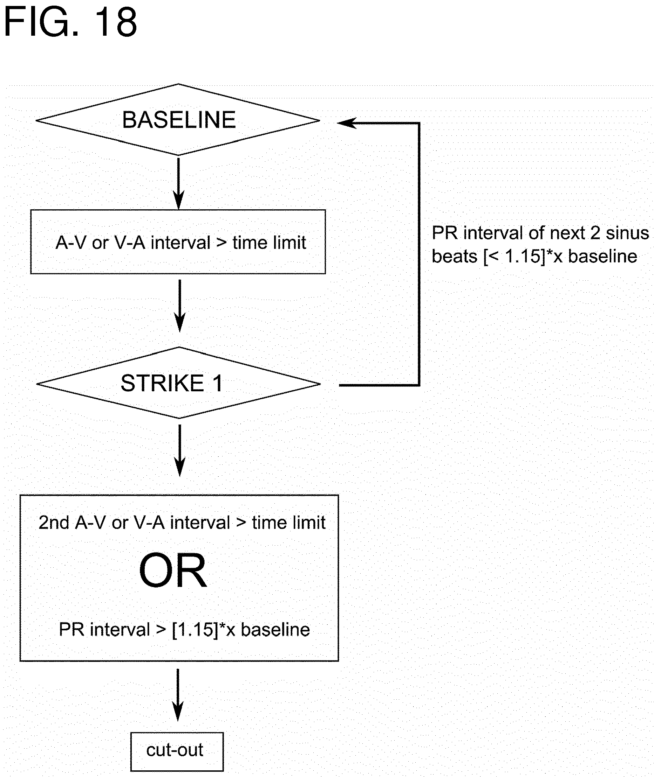

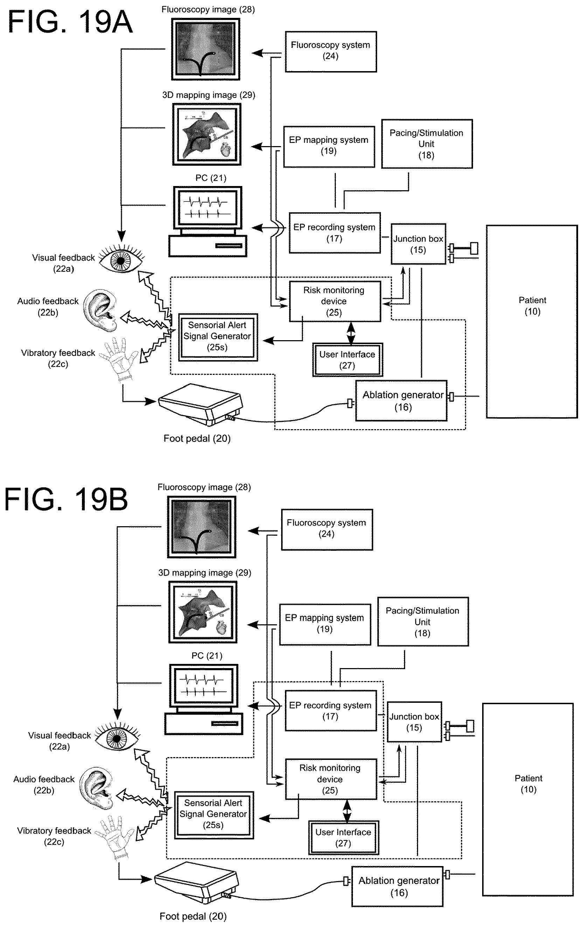

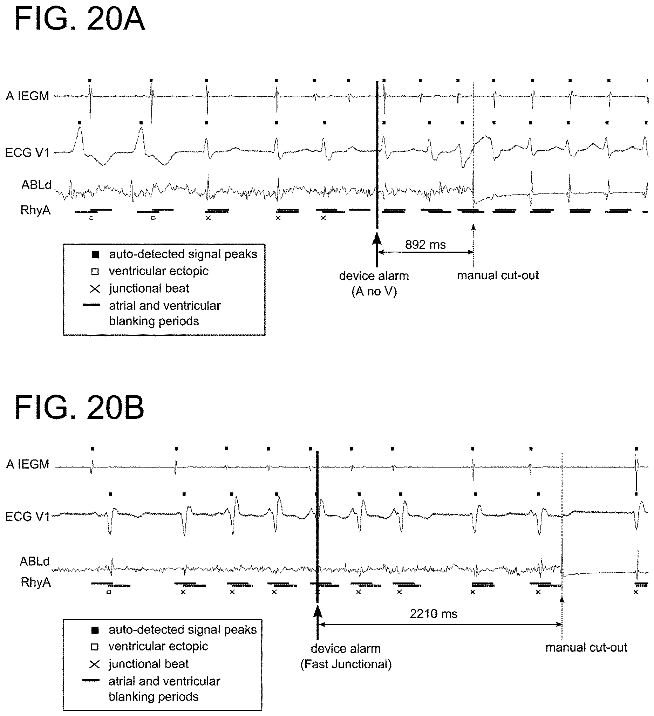

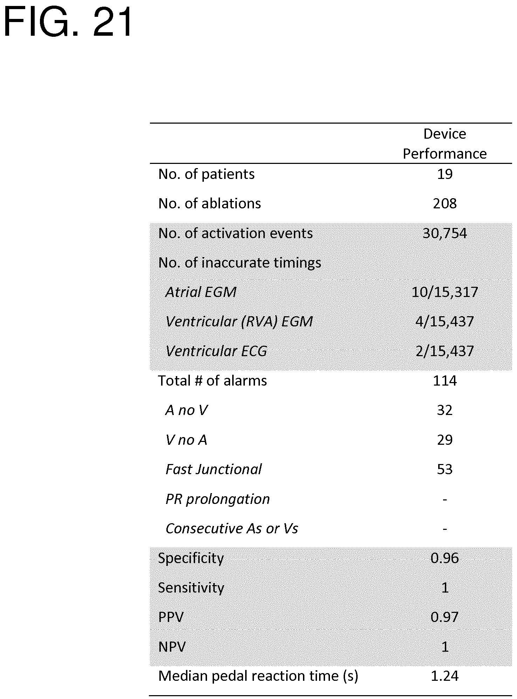

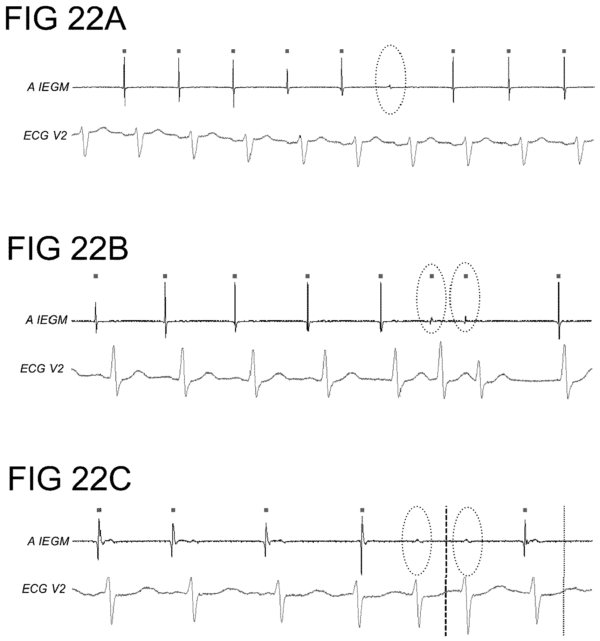

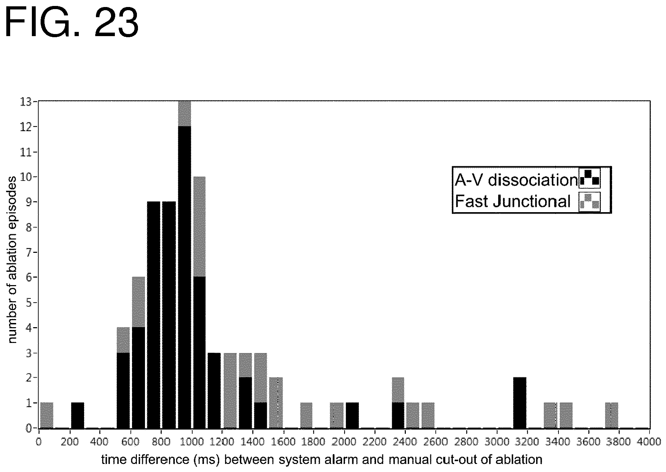

For the purpose of illustrating the invention, there are shown in accompanying drawings, forms which are presently preferred, it being understood that the invention is not intended to be limited to the precise arrangement and intrumentalities shown.
▪ FIG. 1 depicts the general set-up and equipment of the state of the art currently used in a standard cardiac ablation procedure. The "EP" acronym refers to "electrophysiology".
▪ FIG. 2A shows the cardiac anatomy with cut-away surface (shaded in grey) to show the right atrium and right ventricle, being separated by the tricuspid valve. The AV node consists of fast and slow pathway inputs, the compact AV node, and bundle of His. The "IVC" acronym refers to the "Inferior Vena Cava, and the "SVC" acronym refers to the "Superior Vena Cava".
▪ FIG. 2B depicts activation of the heart during normal sinus rhythm, with spread of electrical activation commencing in the sinoatrial node and spreading through the right atrium, and to the right ventricle via the AV node.
▪ FIG. 2C shows a typical body surface electrocardiogram recording, known as "ECG". Atrial depolarisation gives rise to a small deflection known as the 'p' wave 44, whereas ventricular depolarisation gives rise to a much larger amplitude deflection known as the QRS complex 45. The interval between onset of the p wave and QRS complex is known as the PR interval 46, and in the normal heart is at least 120ms in duration.
▪ FIG. 3A depicts activation sequence in the heart when a septal accessory pathway is present that allows antegrade conduction from the atria to the ventricles (so-called Wolff-Parkinson-White syndrome). The electrical impulse is propagated from the atria to the ventricles over both the AV node, and the accessory pathway.
▪ FIG. 3B shows a typical body surface electrocardiogram (ECG) recording from a patient with Wolff-Parkinson-White syndrome, in which the PR interval is shorter (< 120 ms) than normal, and the onset of the QRS complex has a slurred morphology.
▪ FIG. 4A depicts electrical impulse propagation within the heart during atrio-ventricular nodal re-entrant tachycardia (AVNRT), with re-entrant (circular) activation of the AV node leading to near simultaneous atrial and ventricular activation.
▪ FIG. 4B depicts electrical impulse propagation within the heart during atrio-ventricular reciprocating tachycardia (AVRT) utilising a septal accessory pathway, with re-entrant (circular) activation of the AV node and accessory pathway sequentially.
▪ FIG. 5 shows placement of an ablation catheter over the slow input to the AV node for purposes of slow pathway ablation or modification in the treatment of AVNRT. A diagnostic (recording) catheter is placed in the high right atrium for purposes of recording an atrial intracardiac electrogram.
▪ FIG. 6A depicts sinus rhythm during ablation, with electrical impulse propagation proceeding from the sinoatrial node, through the atrioventricular (AV) node, to the ventricles. In the case of normal AV node function, atrial intracardiac electrogram (A IEGM) should proceed the QRS complex of the surface electrocardiogram (ECG) by no more than approximately 250ms (nominal value).
▪ FIG. 6B depicts junctional rhythm during ablation. Electrical activation of the heart originates from the compact AV node or His bundle and spreads outward to both atrial and ventricles simultaneously. In the case of normal AV node function, atrial intracardiac electrogram (A IEGM) should lag the QRS complex of the surface electrocardiogram (ECG) by no more than 130ms (nominal value).
▪ FIG. 6C depicts ventricular ectopic beat (rhythm) during ablation. Electrical activation of the heart originates from the ventricular tissue and spreads retrogradely through the AV node to the atria. In the case of normal AV node function, atrial intracardiac electrogram (A IEGM) should lag the QRS complex of the surface electrocardiogram (ECG) by no more than 250ms (nominal value).
▪ FIG. 7 shows the current invention and its relationship to existing equipment for the purposes of cardiac ablation. The current invention includes a Risk Monitoring Device 25, and a user interface 27, and receives inputs from at least one of a junction box 15 for acquisition of IEGM and ECG, an EP mapping system 19 for transmission of data relating to the 3D location of the ablation catheter tip, and a fluoroscopy unit 24 for evaluation of catheter tip location from low dose x-ray images. The Risk Monitoring Device 25 communicates to the operator via a Sensorial Alert Signal Generator 25s, allowing at least one of a visual, auditory, or vibratory alert to instruct the operator to discontinue ablation.
▪ FIG. 8 shows data on the percentage of ventricular ectopic beats (rhythm), as a function of whether an intracardiac catheter is placed within the right ventricle or not. In 215 ablation episodes from 20 patients in which a ventricular catheter was placed, 36% of ablations had no ventricular ectopic rhythm, whereas 64% had ventricular ectopic rhythm varying between 0.1 to 20% of all beats. In 196 ablation episodes from 20 patients in which a ventricular catheter was not used, ventricular ectopic rhythm was much less, with 95% of ablations having no ventricular ectopic rhythm.
▪ FIG. 9A depicts the phenomenon of indeterminate AV nodal function, due to a sinus beat being closely followed by a ventricular ectopic beat, such that the integrity of AV nodal function cannot be determinated.
▪ FIG. 9B gives an example of a sinus beat being closely followed by a ventricular ectopic beat, which leads to inability to assess AV nodal function (conduction) until the following beat.
▪ FIG. 10A shows a junctional beat with slowed/damaged AV nodal conduction of the electrical impulse to the atria, giving rise to the same intracardiac electrograms as would a ventricular ectopic beat with normal AV nodal conduction (FIG. 10B). The QRS complex on the surface ECG is of narrow morphology and this allows differentiation of the two rhythms.
▪ FIG. 10B shows a ventricular ectopic beat with normal AV nodal conduction of the electrical impulse to the atria, giving rise to the same intracardiac electrograms as would a junctional beat with slowed/damaged AV nodal conduction (FIG. 10A). The QRS complex on the surface ECG is of wide morphology and this allows differentiation of the two rhythms.
▪ FIG. 11A shows a sinus beat with normal AV nodal conduction of the electrical impulse to the ventricles, giving rise to the same intracardiac electrograms as would a sinus beat followed by a ventricular ectopic beat (FIG. 11 B) in which AV nodal conduction is indeterminate. The QRS complex on the surface ECG is of narrow morphology and this allows differentiation of the two rhythms.
▪ FIG. 11B shows a sinus beat followed by a ventricular ectopic beat (in which AV nodal conduction is indeterminate), giving rise to the same intracardiac electrograms as would a sinus beat with normal AV nodal conduction. The QRS complex on the surface ECG is of wide morphology and this allows differentiation of the two rhythms.
▪ FIG. 12A shows an example of atrio-ventricular dissociation, in this case an atrial (A) depolarisation which is not followed by a ventricular (V) depolarisation within 250ms (A no V), indicating risk of continued ablation. A IEGM = atrial intra-cardiac electrogram; ECG = electrocardiogram
▪ FIG. 12B shows an example of atrio-ventricular dissociation, in this case a ventricular (V) depolarisation which is not followed by an atrial (A) depolarisation within 250ms (V no A), indicating risk of continued ablation. A IEGM = atrial intra-cardiac electrogram; ECG = electrocardiogram.
▪ FIG. 12C shows an example of Fast Junctional Rhythm (junctional rhythm exceeding 130bpm), indicating risk of continued ablation. The Risk Index (Ri) is incremented after the 2nd consecutively detected fast junctional beat. A IEGM = atrial intra-cardiac electrogram; ECG = electrocardiogram.
▪ FIG. 12D shows an example of atrio-ventricular dissociation, as evidenced by consecutive atrial (A) depolarisations, without an intervening ventricular depolarisation (consecutive As), indicating risk of continued ablation. A IEGM = atrial intra-cardiac electrogram; ECG = electrocardiogram.
▪ FIG. 12E shows an example of PR interval prolongation exceeding 1.15 x baseline PR interval for 3 consecutive beats, indicating risk of continued ablation. A IEGM = atrial intra-cardiac electrogram; ECG = electrocardiogram.
▪ FIG. 13 shows a schematic of the Risk Monitoring Device's treatment of atrial intracardiac electrogram (IEGM) and ventricular electrocardiogram (ECG) signals, in order to discern cardiac rhythm.
▪ FIG. 14 shows real-time or near real-time display of pulsed signals output from the Risk Monitoring Device to the EP recording system, for the purposes of a visual check that the Risk Monitoring Device is accurately detecting atrial and ventricular depolarisations.
▪ FIG. 15 shows an example of an appropriate user interface on which parameters triggering cut-out of ablation can be set.
▪ FIG. 16 shows an example of the Risk Monitoring Device's computational algorithm employed during ablation of AVNRT.
▪ FIG. 17 shows an example of the Risk Monitoring Device's computational algorithm employed during ablation of AVRT
▪ FIG. 18 shows an example of the Risk Monitoring Device's computational algorithm employed to allow continued ablation despite a single beat of dissociated atrio-ventricular activity.
▪ FIG. 19A depicts incorporation of the Risk Monitoring Device into an existing Ablation Generator (incorporated items enclosed by dashed box in figure)
▪ FIG. 19B depicts incorporation of the Risk Monitoring Device into an existing electrophysiology (EP) Recording System (incorporated items enclosed by dashed box in figure).
▪ FIG. 20A shows the method for determining human operator reaction time to a condition requiring cessation of ablation energy. In this case, ablation was stopped because of an "A no V" condition. The time of manually applied cut-out of ablation (thin vertical dashed line) is derived from the end of ablation-induced artifact (noise) recorded on the distal ablation electrode (ABLd). The time of sensorial alert as determined by the Risk Monitoring Device (thick vertical solid line), called on the figure "device alarm", is subtracted from the manual cut-out time to give 892ms of reaction time. A IEGM = atrial intra-cardiac electrogram; ECG V1 = lead V1 of the electrocardiogram; RhyA = the automated rhythm analysis (refer to the key shown in figure).
▪ FIG. 20B shows the method for determining human operator reaction time to a condition requiring cessation of ablation energy. In this case, ablation was stopped because of "Fast Junctional" rhythm. The time of manually applied cut-out of ablation (thin vertical dashed line) is derived from the end of ablation-induced artifact (noise) recorded on the distal ablation electrode (ABLd). The time of sensorial alert as determined by the Risk Monitoring Device (thick vertical solid line) is subtracted from the manual cut-out time to give 2210ms of reaction time. A IEGM = atrial intra-cardiac electrogram; ECG V1 = lead V1 of the electrocardiogram; RhyA = the automated rhythm analysis (refer to the key shown in figure).
▪ FIG. 21 summarises the results of testing of a prototype of the invention.
▪ FIG. 22A shows an example of under-detection of atrial depolarisation (dashed oval) due to sudden diminution of signal amplitude. A IEGM = atrial intra-cardiac electrogram; ECG V2 = lead V2 of the electrocardiogram.
▪ FIG. 22B shows an example where accurate detection of atrial depolarisations (dashed ovals) is preserved despite sudden diminution of signal amplitude to a slightly lesser degree than FIG. 22A. A IEGM = atrial intra-cardiac electrogram; ECG V2 = lead V2 of the electrocardiogram.
▪ FIG. 22C shows an example where accurate detection of atrial depolarisations is preserved despite the presence of ventricular far-field signal (dashed ovals) on the atrial recording. The far field signal is appropriately not detected as atrial depolarisations. A IEGM = atrial intra-cardiac electrogram; ECG V2 = lead V2 of the electrocardiogram.
▪ FIG. 23 shows the distribution of human reaction times determined during prototype testing. Reaction time improvement is the time difference between human (manual) cut-out of ablation, and automatic sensorial alert provided by the Risk Monitoring Device. Cut-outs were for both atrio-ventricular and ventriculo-atrial dissociation (grouped together as A-V dissociation; solid bars), and for Fast Junctional rhythm (grey bars).

### DETAILED DESCRIPTION

The following description is of the best mode presently contemplated for carrying out the invention. This description is not to be taken in a limiting sense, but is made merely for the purpose of describing the general principles of the invention. The scope of the invention should be made with reference to the claims.

### EQUIPMENT FOR ELECTROPHYSIOLOGY STUDY AND ABLATION

For the purposes of explaining the current applicant's system, a common setup of equipment for the purposes of electrophysiologic (EP) study and ablation is first reviewed in more detail (FIG. 1).

Catheters are placed inside of the heart 11 usually via the femoral veins. A minimum of two catheters is generally required to diagnose arrhythmia mechanism.

### Diagnostic Cathether

One or more diagnostic catheters 13 are placed to allow both recording of intracardiac electrograms, and programmed stimulation of the heart. Diagnostic catheters are typically connected to junction box 15 which feeds signals to an electrophysiology (EP) recording system 17.

### ECG (body surface)

Voltage signals are also recorded from the body surface in the form of an electrocardiogram (ECG) 14, typically of 12 leads in standard locations (only 3 leads shown in FIG. 1).

### Ablation Catheter

An ablation catheter 12 is used to direct ablation energy to specific areas of the heart found to be critical to either arrhythmia triggering or maintenance.

### Ablation generator

The ablation generator unit 16, which connects to the ablation catheter, provides the energy for ablation. It is one example of the power source of the ablation catheter 12. This may be in the form of radiofrequency (RF) current, cryo (freezing), high intensity focused ultrasound (HIFU), microwave energy, or other forms; RF being the most commonly employed presently.

Examples of current commercially available RF generators include :
▪ EP-Shuttle and SmartAblate^{™} (Stockert),
▪ Ampere^{™} (St. Jude Medical),
▪ Maestro 3000^{™} (Boston Scientific).

The ablation generator can usually be controlled (that is to say, ablation energy commenced and terminated) by a switch on the unit, or, by a foot pedal 20.

### EP recording system

The EP recording system 17 is used to condition (amplify and filter) the intracardiac and ECG signals, convert these to digital form, and display these to the operator and technical staff on a PC monitor(s) 21, for the purposes of performing the procedure.

Examples of current commercially available EP recording systems include :
▪ BARD@ LABSYSTEM™ PRO EP (Boston Scientific),
▪ Sensis^{™} (Siemens),
▪ EP-WorkMate^{™} (St. Jude Medical),
▪ CardioLab^{™} (GE Healthcare).

### EP mapping system

The EP mapping system 19 may also be used to condition and display intracardiac and ECG signals, but also performs the function of displaying three-dimensional catheter position relative to a reconstructed cardiac geometry, which augments standard x-ray based fluoroscopic methods of catheter navigation.

Examples of current commercially available EP mapping systems include :
▪ Carto^{™} (Biosense Webster),
▪ Rhythmia^{™} (Boston Scientific),
▪ EnSite^{™} and Velocity^{™} Cardiac Mapping Systems (St. Jude Medical).

### Pacing/ stimulation Unit

The Pacing/Stimulation Unit 18 provides current pulses to stimulate the heart at programmed rates and patterns, to assist in the diagnosis of arrhythmia mechanism.

Examples of current commercially available Pacing/Stimulation Units include:
- StimCor^{™} (Boston Scientific).
- Micropace (Micropace EP Inc).

Variations of the above connection configurations can be implemented according to various embodiments of the invention. The figure 1 represents one configuration of connectivity of items that may be implemented according to the invention.

For example, it is possible on some commercially available systems to plug all catheters directly into the EP mapping system bypassing the junction box, with the EP recording system, Pacing/Stimulation Unit, and ablation generator being slaved off the EP mapping system. Many other configurations are feasible, while still maintaining overall connectivity of the equipment items shown in FIG. 1. Moreover, it is possible to integrate items in FIG. 1 into combined systems, for example, the Pacing/Stimulation Unit may be integrated into an EP recording system, as in, for example, the commercially available Claris™ EP recording system (St Jude Medical).

### PROCEEDING TO AN ABLATION CLOSE TO THE AV NODE

Using the above described set-up, ablation of cardiac tissue lying close to, or adjacent to, the AV node, is possible, but carries a risk of damage to the compact AV node 31 or His bundle 30, leading to permanent AV block, wherein there is failure of propagation of the electrical impulse from the atria to the ventricles, and co-ordination between atrial and ventricular contraction is lost.

Because permanent heart block generally requires implantation of a permanent pacemaker, this is considered a major complication, and may reduce the quality and quantity of the patient's life.

The invention allows prediction of occurrences of AV block by analysing recorded signals and ablation catheter tip location.

### Atrio-ventricular and ventriculo-atrial dissociation

AV block may be heralded by changes seen in the cardiac signals displayed on the EP recording system monitor 21. Specifically, the occurrence of transient dissociation of atrial and ventricular depolarisation may indicate impending heart block, and can be detected by analysis of the intracardiac atrial electrogram IEGM together with the body surface ECG, wherein each atrial or ventricular signal is not associated with a counterpart ventricular or atrial signal within a defined time period as illustrated in figures 12A and 12B.

### Fast junctional rhythm, consecutive atrial or ventricular depolarisations, PR interval prolongation

In addition, fast junctional rhythm, as illustrated in figure 12C, consecutive atrial or ventricular depolarisations, as illustrated in figure 12D, or PR interval prolongation, as illustrated in figure 12D, may indicate impending heart block.

### Visual checking

When ablating close to the AV node, the operator and technical assistant(s) closely watch the signals on the monitor 21, and if any of the aforementioned conditions arise, ablation is terminated manually be pressing on a switch on the ablation unit, or, alternatively by releasing the foot pedal 20. If a foot pedal is used, this may be controlled directly by the operator, or, by a technician. Human recognition of conditions indicating impending heart block, and reaction to said recognition takes typically 1-4 seconds. This is due to the inherent limitations of speed and consistency of human reaction, and because there are competing needs to visually monitor both catheter location and cardiac rhythm; each of these tasks detracting from the other. The process of human visual feedback 22 therefore suffers from significant limitations. Any delay in reaction to the markers of risk of heart block may cause irreversible damage to tissue and render heart block more likely to occur.

### AUTOMATED CUT-OFF OF ABLATION

The invention comprises a system for supplementing human visual feedback 22 with automated assessment of at least one of cardiac rhythm and catheter position, allowing automatic generation of a sensorial alert when conditions indicating risk of heart block occur. The system disclosed is able to react to risk conditions faster than the human reaction, and in a more consistent manner.

In the method and system of this invention, cardiac rhythm analysis is performed with use of one or more atrial intracardiac electrogram signal IEGM, and the use of one or more body surface ECG signal.

### Junction box

In preferred embodiments of the invention, these signals are split from the Junction Box 15 shown in figure 7 into the Risk Monitoring Device 25, or alternatively are derived from connection between the Risk Monitoring Device 25 and the EP recording system 17 or EP mapping system 19. Other specific connectivities are possible, for example direct catheter connection to the Risk Monitoring Device 25 bypassing the Junction Box 15, and remain within the scope of the invention. The Risk Monitoring Device conditions the signals by amplification, filtering, and analog-to-digital conversion. Alternatively, partially or fully preconditioned signals may be obtained from the EP recording system 17 or EP mapping system 19 and input to the Risk Monitoring Device 25.

### Timing and Morphology analysis

The Risk Monitoring Device performs real-time or near real-time computational analysis of signals in order to derive timing of atrial and ventricular depolarisation, in the former based on peak or onset of atrial electrogram deflection, and in the latter based on peak or onset of ECG QRS complex deflection as shown in figure 13.

Analysis of ECG QRS complex morphology is also performed to determine the nature of ventricular depolarisation, specifically, whether ventricular depolarisation arises from a junctional or ectopic beat, and whether ventricular depolarisation is exclusively via the AV node, or pre-excited from an accessory pathway.

Where there is conflict in the assessment of timing and morphology across leads of the ECG, a voting algorithm may be employed to determine a majority decision as shown in figure 13.

### Timing analysis

In a first embodiment, timing analysis may be performed with a threshold of signal magnitude and/or derivative, with the threshold fixed to a percentage of the preceding n^{th} number of signal peaks.

In a second embodiment, an alternate method may employ signal phase analysis (for example utilising a Hilbert's transform) with signal timing defined by a zero crossing of phase.

### Morphology analysis

In one embodiment, morphology analysis may be performed using auto-correlation based matching of the real-time QRS complex with a template QRS, the template QRS having been automatically stored from the same patient at procedure onset, prior to ablation.

In a second embodiment, additional morphology analysis methods may employ cross-correlation of QRS signal with generic (derived from other individuals) template signals from normal and ectopic beats, as well as from other conditions causing change to QRS morphology such as impaired conduction in the Purkinje fibre branches 41 (so-called "bundle branch block").

Yet further methods may employ determination of QRS complex width, and QRS signal positive/negative deflection sequence.

In a preferred embodiment of the invention, the Risk Monitoring Device 25 outputs pulsed (square wave) signals to the EP recording system 17, via connection to the Junction Box 15 (FIG. 7), to mark the timings of atrial and ventricular depolarisations, as determined by the Risk Monitoring Device. As shown in FIG. 14, these pulses are thus able to be displayed on the PC 21 in real or near-real time, in order to provide a visual check that the Risk Monitoring Device is functioning correctly.

### Examples

An example of a suitable data acquisition (DAQ) card capable of fulfilling the requirements of the Risk Monitoring Device 25 is the sbRIO-9639 (National Instruments, NI). Similar DAQ cards from the same or other manufacturers could be employed. Processing of atrial IEGM and ECG signals can performed in real-time on the microprocessor or the FPGA of the NI sbRIO-9639. Testing of the applicant's prototype has been performed using the aforementioned NI sbRIO-9639, with software written in LABVIEW®. Use of other programming languages or methods of data acquisition are considered within the scope of the invention.

### SENSORIAL ALERT MECHANISM

Because automated cut-out of ablation in response to markers of risk of heart block could lead to machine-induced error and patient harm, the preferred embodiment of the invention is for the Risk Monitoring Device 25 to generate a sensorial alert, to which the operator and assistance(s) can react to terminate ablation energy.

However, the invention may be combined in one embodiment with an automated cut-out of ablation when the conditions indicating risk of AV block are detected. For instance, a first threshold may be defined as a fist risk index, a second threshold may defined as a second risk index which is higher than the first risk index. A third threshold which is higher than the second risk index may be defined in order to automatically engage cut out of the catheter energy when the consequence of the ablation lead to a high risk of heart block.

The invention allows defining different strategies for alerting an operator including stopping the ablation when the risk index is superior to a third threshold.

A preferred embodiment of the invention includes sensorial alerts that combine at least one of a audio, visual, and vibratory alert. In respect of the audio alert, the volume and tone of the alert can be set to indicate the level of risk of continued ablation. Alternatively, a binary audio alert, for example a "beep", could be set to instruct the operator to stop ablation. In respect of the visual alert, the colour, size, and pattern of the alert, displayed on, for example, an LCD screen, is used to indicate to the operator the degree of risk. In respect of the vibratory alert, the intensity or amplitude, and pattern of vibration can be set in order to indicate to the operator the degree of risk. A preferred embodiment includes the incorporation of a vibratory alert into the handle of the ablation catheter, so that it can be felt directly through the operator's hand.

In the case that the operator elects to continue ablation despite being alerted to a risk of heart block, he/she holds responsibility for the decision to ablate, having taken into consideration the safety of continued ablation, and if necessary, repositioned the catheter to a safer location.

In one embodiment, the Risk Monitoring Device is able to give feed-back on the reaction time of the operator (releasing the foot pedal) to the sensorial alert.

### USERINTERFACE

The Risk Monitoring Device is connected to a user interface 27, as represented in figure 7, which allows operator-led adjustment of the parameters causing automated sensorial alert.

An example of a suitable user interface is shown in figure 15.

For example, whether an alert occurs for atrio-ventricular dissociation, as shown in figures 12A, 12B, and 12D, can be selected by the "Blocked Beats Cut-off" knob as shown in figure 15.

Whether an alert occurs for PR interval prolongation as shown in figure 12E and at what degree of prolongation, 115% or 125%, can be selected using the "PR Prolongation" knob as shown in figure 15.

The heart rate causing cut-out, if any, for Fast Junctional Rhythm as shown in figure 12C can be selected using the "Junctional Rate Cut-off" knob as shown in figure 15.

Other more 'advanced settings' can also be adjusted, for example the maximum delay allowed between atrial and ventricular depolarisations, called the A-V interval, nominally set to 250ms.

The specific alert conditions set will depend on the type of ablation being performed, the particular patient's physiology, and individual operator preference. For example, a patient with pre-existing slow / diseased conduction of electrical impulse from atria to the ventricles, so-called first degree heart block, may need allowance of longer A-V intervals compared to a patient with normal baseline atrio-ventricular conduction. Similarly, an operator with many years of experience in ablation may chose less conservative settings on the device compared to an operator with less experience.

### DEVICE ALGORITHMS

There may be differences in the computational algorithms employed by the Risk Monitoring Device 25 for different arrhythmia, to allow for safe ablation in each condition.

In the case of AVNRT, a marker of successful ablation is the development of slow junctional rhythm, as this indicates some degree of heating to the AV node, without risk of damage to the compact node or His bundle components. However, fast junctional rhythm (exceeding ∼130bpm) is to be avoided.

In contrast, in the case of AVRT or ablation of an accessory pathway close to the AV node, no junctional rhythm is desirable whilst the ventricle is pre-excited via the accessory pathway. This is because integrity of AV nodal conduction is uncertain during junctional rhythm with pre-excitation. Algorithms that reflect these differences in strategy are employed on the Risk Monitoring Device.

The algorithm currently preferred for use in slow pathway ablation for AVNRT is shown in figure 16.

The algorithm currently used for accessory pathway ablation during pre-excitation of the ventricle is shown in figure 17.

The main differences between algorithms relates to the response to junctional rhythm and PR interval change.

In the case of accessory pathway ablation as shown in figure 17 :
▪ sensorial alert may occur for the presence of any junctional beats while the heart remains in pre-excited rhythm (because integrity of AV nodal conduction is unable to be assessed while in junctional rhythm), and;
▪ sensorial alert for PR prolongation is not enabled until pre-excitation is lost by successful ablation of the accessory pathway, to avoid unnecessary cut-out at the instant when conduction over the accessory pathway is extinguished.

An additional algorithm may also be employed that allows continued ablation without an alert despite the occurrence of a single dissociated beat, as for instance a single A without V, or a single V without A, corresponding to the cases illustrated in figures 12A and 12B, yet generates an alert in response to multiple dissociated beats.

This algorithm is shown in figure 18, and is intended as an "advanced" mode of ablation, to be used for ablation in patients with a high number of non-conducted ectopic beats, or for operators of sufficient skill that cut-out of ablation for a single non-conducted beat is deemed unnecessary. The algorithm shown in figure 18 is based around requirements of two "strikes" for the device to alarm :
▪ the first strike being for a non-conducted (dissociated) beat, and ;
▪ the second strike being for a second non-conducted beat, or, PR interval prolongation.

The first strike is able to be redeemed by the subsequent occurrence of two consecutive sinus beats of normal PR interval.

The algorithms displayed in figures 16, 17, and 18 are included for indicative purposes only and are not to be taken in a limiting sense with respect to the scope of the invention; many variations on these algorithms are possible. The algorithms given are particular to ablation in sinus rhythm. Further algorithms may also be employed for ablation during tachy-arrhythmia.

### INTEGRATION TO EXISTING EQUIPMENT

In one embodiment of the invention, the Risk Monitoring Device 25 may be a stand-alone piece of equipment. In other embodiments, it could be variously incorporated into an ablation generator 16, as shown in figure 19A, an EP recording system 17, as shown in figure 19B, or an EP mapping system 19, (not shown).

In the case of incorporation into other items of equipment as described above, the Sensorial Alert Signal Generator 25s may also be incorporated into the circuitry and/or housing of the same item of equipment.

Equally, in the case of incorporation into other systems or devices, there may not be a requirement for a stand-alone user interface monitor 27. Rather, the functionality of the user interface 27 could be added to existing user interfaces associated with the EP recording system, the EP mapping system, or the ablation generator.

### ABLATION ENERGY TYPES

The present invention can be equally used with any form of ablation energy, including but not limited to radiofrequency (RF) ablation, cryo-ablation, high intensity focused ultrasound (HIFU) ablation, and microwave ablation.

### PROTOTYPE TESTING

A prototype has been developed using the preferred strategy outlined above, and tested on signals acquired from 19 patients (208 ablation episodes) during the course of AVNRT ablation via slow pathway ablation or modification. Intracardiac atrial electrograms recorded from either the high right atrium (HRA), or the coronary sinus (CS), were input to the Risk Monitoring Device 25, along with three leads from the standard ECG (leads I, V1, V4).

Activity in the atrium and ventricles was assessed using EGM and ECG peak detection, by way of voltage magnitude and 1st derivative adaptive thresholding as described above, incorporating a threshold decay function to allow resumption of detection after lead or catheter disconnection. Subsequent rhythm determination was by rule-based analysis, incorporating QRS complex morphology matching to distinguish junctional from ventricular ectopic beats. Post detection blanking intervals of 180ms and 200ms were applied to the atrial and ventricular signals respectively, to prevent double counting of activations. No blanking period was applied to the atrial signal following ventricular activation, to ensure that junctional rhythm could be adequately tracked. Cardiac rhythm was arbitrarily classified as 'sinus' where there was sequential atrial and ventricular depolarisation separated by ≥ 90 ms, 'ventricular' where there was sequential ventricular and atrial depolarisation separated by ≥ 90 ms, and 'junctional' where atrial and ventricular depolarisations were within 90ms of each other.

The algorithm shown in figure 16 was programmed in the graphical language LabViewTM 12.0 (National Instruments; ni.com), and implemented on a National Instruments Single-board RIO microprocessor. The nominal settings that would trigger a sensorial alert were :
▪ A-V conduction time exceeding 250 ms, and V-A conduction time exceeding 250ms for a ventricular ectopic beat, or 130ms for a junctional beat,
▪ junctional rhythm > 130 bpm, sustained for 3 consecutive beats,
▪ PR interval prolongation > 1.15x pre-ablation baseline, sustained for 3 beats, and
▪ two consecutive atrial or ventricular depolarisations.

The timing of individual atrial and ventricular depolarisations (total of 30,754 EGM and ECG complexes), and the cardiac rhythm at each beat, was assessed manually by an experienced clinician, to act as a gold-standard comparison to the algorithm. Database signals were fed to the device with real-time sampling to simulate a 'live' electrophysiologic study. The device signal interpretations and alarm conditions were stored and later analysed.

Alarm conditions of the device were compared to the clinical cut-outs of ablation, which were with use of a foot pedal controlled RF generator (StockertTM, Biosense Webster). The foot pedal was controlled by a Physiologist instructed to terminate ablation in response to :
▪ a single event of failed or delayed atrio-ventricular conduction, or a single event of failed or delayed ventriculo-atrial conduction, or ;
▪ junctional rhythm judged to be visually faster than ∼130 bpm.

In rare cases, the Physiologist was instructed to continue ablation despite fast junctional rhythm, or the onset of AVNRT. The time of pedal release was able to be determined from the signal recorded at the distal bipole of the ablation catheter, which displayed an electrical artifact at the time of cut-out. This allowed comparison of response speeds of human and device in all cases, with examples of A no V cut-out and Fast Junctional cut-out given in Figures 20A and 20B respectively.

The invention prototype performance is summarised in figure 21. The device assigned accurate times to atrial depolarisations in all but 10 / 15,317 cases, and to ventricular depolarisations in all but 2 / 15,437 cases. The inaccurate timings occurred in 3 patients. Of the 10 inaccurate EGM timings, 8 were due to under-detection of small amplitude EGM signals during periods of sudden diminution of signal amplitude (example given in figure 22A, with an example of successful detection in similar circumstances given in figure 22B). In two recordings, inaccurate assignment was due to sudden large amplitude fluctuation in EGM baseline, caused by patient movement. There were no cases of over-detection of ventricular far-field signal on the atrial EGM (example in figure 22C). The two cases of inaccurate timing assignment in ECG peak were due to the well described phenomenon of T wave over-sensing.

Of the 208 ablation episodes, the device triggered an alarm in 114. Of these, 53 alarms were for detection of fast junctional rhythm, 32 in response to failed atrio-ventricular conduction, and 29 in response to failed ventriculo-atrial conduction as represented in figure 21. There were three false positive alarms which occurred immediately following inaccurate assignment of atrial or ventricular depolarisation time during ablation (specificity of device termination = 96%, PPV 97%). There were no instances where the device missed a termination condition (sensitivity = 100%, NPV 100%).

For the 111 appropriate device alarms, response speed of the device was compared to the speed of manual RF foot-pedal release. The device performed a median of 1,238 ms faster (range 14 to 132,919) than the human response. In no cases did the device lag human response. The distribution of response time differences (manual cut-out time minus device alarm time) is shown in figure 23, for episodes where this time difference was < 4s (n= 79; median = 971 ms). Reaction time differences longer than 4s were assumed to be due to intentional continuation of ablation.

### SUMMARY

We have described an invention and system for the automated detection of risk of AV block during ablation near to the AV node, with automatic sensorial alert/alarm transmitted to the operator to allow more rapid and consistent termination of ablation energy. The present invention may be embodied in other specific forms without departing from the spirit or essential attributes thereof. It is therefore desired that the present embodiment be considered in all aspects as illustrative and not restrictive, reference being made to the appended claims rather than to the foregoing description to indicate the scope of the invention.

## Claims

1. A monitoring system for ablation of cardiac tissue in close proximity to the AV node of the heart, **characterized in that** it comprises :
▪ At least one ablation catheter (12) powered by a power source (16);
▪ At least one electrode (13) acquiring an intracardiac electrogram (IEGM), called IEGM electrode ;
▪ At least one set of electrodes (14) acquiring a surface electrocardiogram (ECG), called ECG electrodes ;
▪ At least one calculator (15, 16, 17, 18, 19, 25, 27) and a memory for measuring in real time at least one of (i) signals acquired from the IEGM electrode and the ECG electrodes, and (ii) catheter tip location, in order to automatically generate an estimate of the level of risk of heart block, called Risk index (Ri), said risk index (Ri) activating a sensorial alert.

2. A monitoring system according to claim 1, **characterized in that** the sensorial alert is at least one of a visual signal, an audio signal or a vibratory signal emitted by a sensorial alert signal generator (25s).

3. A monitoring system according to claim 2, **characterized in that** the visual, audio, or vibratory signals is activated with synchronised or sequential timing according to the generation of the Risk Index (Ri).

4. A monitoring system according to claims 2 and 3, **characterized in that**:
▪ At least one of frequency and volume of the sound of the audio signal depends on the level of the Risk Index (Ri) and/or;
▪ At least one of the colour, size and pattern of the visual signal depends on the level of the Risk Index (Ri) and/or;
▪ At least one of the amplitude and pattern of vibration of the vibration signal depends on the level of the Risk Index (Ri).

5. A monitoring system according to any of claims 2 to 4 **characterized in that** the vibratory signal is incorporated into an ablation catheter handle.

6. A monitoring system according to any of claims 1 to 5, **characterized in that** at least one calculator (15, 16, 17, 18, 19, 25, 27) and a memory is used for measuring in real time a displacement of the ablation catheter (12) in order to automatically modify the risk index (Ri) when detecting a displacement of the ablation catheter (12) exceeding a predefined limit.

7. A monitoring system according to any of claims 1 to 6, **characterized in that** real time displacement of the ablation catheter (12) away from the desired ablation location is determined from at least one of (i) catheter localisation based on object tracking in fluoroscopic low-dose x-ray images, or (ii) three-dimensional catheter tip position obtained from measurement of impedance and/or magnetic field at the catheter such as performed in an EP mapping system (19).

8. A monitoring system according to claim 1, **characterized in that** the calculator comprises a signal timing function in order to derive timing of atrial and ventricular depolarisation, the atrial depolarisation being measured by the IEGM electrode (13), and the ventricular depolarisation being measured by the ECG electrodes (14).

9. A monitoring system according to claim 8, **characterized in that** the signal timing function compares the delay between atrial and ventricular depolarisations to a minimum predefined threshold of time and/or a maximum predefined threshold of time, the signal timing function being configured for generating a cardiac risk indicator when said measured delay exceeds at least one of the predefined thresholds.

10. A monitoring system according to claim 8, **characterized in that** the signal timing function comprises a first voting function allowing the determination of timing when more than one IEGM electrode or more than one ECG electrode is used.

11. A monitoring system according to any of claims 1 to 10, **characterized in that** the calculator comprises a signal morphology function in order to determine the nature of the ventricular depolarisation by digitally comparing the QRS complex acquired by the ECG electrodes (14) with a template QRS stored in memory.

12. A monitoring system according to any of claims 8 to 11, **characterized in that** the calculator is configured to determine in real-time or near real-time the cardiac rhythm using at least a return value of the signal timing function and/or a return value of the signal morphology function.

13. A monitoring system according to any of claims 1 to 12, **characterized in that** the calculator comprises a mapping function allowing automatic tracking of the location of the tip of the ablation catheter (12) in three-dimensional space, said location of the tip being determined by a electrophysiology (EP) mapping system (19) in real time or near real time, or by low-intensity x-ray (fluoroscopy) of the ablation catheter in real time or near real time.

14. A monitoring system according to any of claims 1 to 13, **characterized in that** the cardiac risk index (Ri) is a probability of AV block occurrence, said probability being **characterised by**:
• Cardiac rhythm comprising at least one occurrence of:
i. an atrio-ventricular interval prolongation ;
ii. a ventriculo-atrial interval prolongation ;
iii. an atrio-ventricular dissociation ;
iv. a ventriculo-atrial dissociation ;
v. a fast junctional rhythm ;
vi. a PR interval prolongation ;
vii. consecutive atrial depolarisations ;
viii. consecutive ventricular depolarisations ;
ix. indeterminate atrio-ventricular nodal conduction,
• Catheter tip displacement exceeding a pre-determined threshold away from the initial ablation position.

15. A monitoring system according to any of claims 1 to 14, **characterized in that** it comprises a user interface allowing the selection of:
▪ a first set of parameters for processing the signal timing function and/or;
▪ a second set of parameters for processing the signal morphology function and/or;
▪ a third set of parameters for processing the mapping function and/or;
▪ a fourth set of parameters for defining the cardiac rhythms of risk which will be used to determine the risk index (Ri) ;
▪ a fifth set of parameters for defining the ablation catheter (12) displacement limit which will be used to determine the risk index (Ri).

16. A monitoring system according to any of claim 1 to 14, **characterized in that** it utilizes at least one calculator and memory incorporated into at least one existing system used for the performance of cardiac ablation, said existing systems inclusive of but not limited to :
• a pacing unit (18) that provides current pulses at programmed rates and patterns for stimulating the heart;
• a junction box (15) that provides a common interface gathering the connections of ECG electrodes and IEGM electrodes in order to deliver acquired signals to the calculator and existing systems ;
• an electrophysiology (EP) recording system (17) that records and displays electrical signals from the heart for the purposes of diagnosis and treatment of arrhythmia ;
• an electrophysiology (EP) mapping system (19) that records and displays the three-dimensional location of catheters ;
• a sensorial alert signal generator (25s) that generates a sensorial signal when a calculator determining a value of a risk index beyond a predefined threshold;
• an ablation generator (16) that provides power to the ablation catheter.
